(19) Europäisches Patentamt
European Patent Office
Office européen des brevets

(11) Publication number: 0 106 515 B1

(12) EUROPEAN PATENT SPECIFICATION

(45) Date of publication of patent specification: 28.12.88

(21) Application number: 83305278.0

(22) Date of filing: 09.09.83

(51) Int. Cl.[4]: C 07 D 249/08, C 07 D 401/06, C 07 D 401/12, C 07 D 405/06, C 07 D 405/14, A 61 K 31/41

# (54) Triazole anti-fungal agents.

(30) Priority: 30.09.82 GB 8227978
02.02.83 GB 8302888
07.05.83 GB 8312624

(43) Date of publication of application: 25.04.84 Bulletin 84/17

(45) Publication of the grant of the patent: 28.12.88 Bulletin 88/52

(84) Designated Contracting States: AT BE CH DE FR GB IT LI LU NL SE

(56) References cited:
EP-A-0 046 633
EP-A-0 055 833
EP-A-0 060 223
EP-A-0 084 236
EP-A-0 115 400
GB-A-2 120 235

**The file contains technical information submitted after the application was filed and not included in this specification**

(73) Proprietor: Pfizer Limited
Ramsgate Road
Sandwich Kent CT13 9NJ (GB)
(84) GB

(73) Proprietor: Pfizer Corporation
Calle 15 1/2 Avenida Santa Isabel
Colon (PA)
(84) BE CH DE FR IT LI LU NL SE AT

(72) Inventor: Richardson, Kenneth, Dr.
48 St. Stephens Hill
Canterbury Kent (GB)
Inventor: Gymer, Geoffrey Edward, Dr.
19 Palmer Road Wingham
Canterbury Ken (GB)
Inventor: Cooper, Kelvin, Dr.
36 Newington Road
Ramsgate Kent (GB)

(74) Representative: Wood, David John et al
Pfizer Limited Ramsgate Road
Sandwich Kent CT13 9NJ (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to novel triazole derivatives which have antifungal activity and are useful in the treatment of fungal infections in animals, including humans. EP—A—46633 and EP—A—55833 disclose certain triazole derivatives having antifungal properties.

According to the invention, there are provided compounds of the formula:—

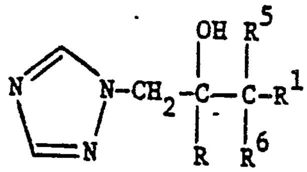 --- (I)

where

R is phenyl optionally substituted by 1 to 3 substituents each independently selected from F, Cl, Br, I, $CF_3$, $C_1$—$C_4$ alkyl and $C_1$—$C_4$ alkoxy, or R is a 5-chloropyrid-2-yl group;

$R^1$ is —CN, —$CSNH_2$, or —$CONR^2R^3$ where either

a) $R^2$ is H or $C_1$—$C_6$ alkyl, and $R^3$ is H, $C_1$—$C_6$ alkyl, benzyl, phenethyl, phenyl, —$CH_2CF_3$, adamantyl, pyridylmethyl, $C_3$—$C_7$ cycloalkyl, carbamoylmethyl, ($C_2$—$C_4$ alkenyl)methyl, 2-hydroxyethyl, 2-(dimethylamino)ethyl, 2-(methylthio)ethyl, 2-(methylsulphinyl)ethyl, 2-(methyl- sulfonyl)ethyl or 2-phenoxyethyl; said benzyl, phenethyl, phenyl and phenoxy groups being optionally ring-substituted by 1 or 2 substituents each independently selected from $C_1$—$C_4$ alkyl, $C_1$—$C_4$ alkoxy, F, Cl, Br, I and $CF_3$,

(b) $R^2$ and $R^3$ taken together with the nitrogen atom to which they are attached represent a group of the formula:—

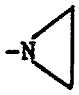 , 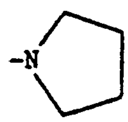 , 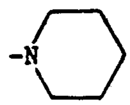 , 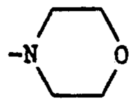 or 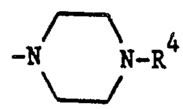

where

$R^4$ is H, $C_1$—$C_4$ alkyl, $C_2$—$C_4$ alkanoyl or ($C_1$—$C_4$ alkoxy)carbonyl;

c) $R^2$ is H or $C_1$—$C_4$ alkyl and $R^3$ is $C_2$—$C_4$ alkanoyl or benzoyl, said benzoyl group being optionally substituted by 1 or 2 substituents each independently selected from F, Cl, Br, I, $CF_3$, $C_1$—$C_4$ alkyl and $C_1$—$C_4$ alkoxy); or

(d) $R^2$ and $R^3$ are both ($C_1$—$C_4$ alkoxy)carbonyl;

$R^5$ is H or $CH_3$; and

$R^6$ is H or $CH_3$;

and their pharmaceutically agriculturally acceptable salts.

The invention further provides a pharmaceutical composition comprising a compound of the formula (I), or a pharmaceutically acceptable salt thereof together with a pharmaceutically acceptable diluent or carrier.

The invention further provides a compound of the formula (I), or a pharmaceutically acceptable salt thereof, for use as a medicament.

In one aspect R is preferably phenyl substituted by 1 to 3 substituents, more preferably 1 or 2 substituents, each independently selected from F, Cl, Br, I and $CF_3$.

R is preferably 4-fluorophenyl, 4-chlorophenyl, 4-bromophenyl, 4-iodophenyl, 4-trifluoromethylphenyl, 2-chlorophenyl, 2,4-dichlorophenyl, 2,4-difluorophenyl, 2,5-difluorophenyl; 2-fluoro-4-chlorophenyl, 2-chloro-4-fluorophenyl, 2,4,6-trifluorophenyl and 4-bromo-2,5-difluorophenyl.

R is more preferably 2,4-dichlorophenyl, 4-chlorophenyl or 2,4-difluorophenyl.

R is most preferably 2,4-dichlorophenyl or 2,4-difluorophenyl.

$R^1$ is preferably —CN, —$CSNH_2$, or $CONR^2R^3$ whether either

(a) $R^2$ is H, $CH_3$ or $C_2H_5$, and $R^3$ is H, $C_1$—$C_6$ alkyl, p-chlorobenzyl, p-chlorophenethyl, p-methylphenethyl, —$CH_2CF_3$, 1-adamantyl, 4-pyridylmethyl, cyclopropyl, carbamoylmethyl, —$CH_2.CH{=}CH_2$, 2-hydroxyethyl, 2-(dimethylamino)ethyl, 2-(methylthio)ethyl, 2-(methylsulphinylethyl), 2-(methylsulphonylethyl), p-chlorophenyl, or 2-(p-chlorophenoxy)ethyl;

(b) $R^2$ and $R^3$ taken together with the nitrogen atom to which they are attached represent a group of the formula:—

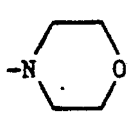 , 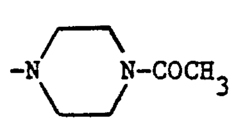 , 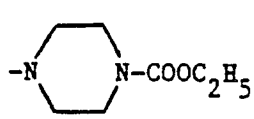 , 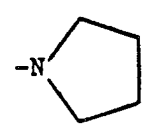 or 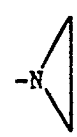 ;

(c) $R^2$ is H and $R^3$ is acetyl, propionyl or p-chlorobenzoyl; or

(d) $R^2$ and $R^3$ are both —$COOCH_3$.

$R^1$ is more preferably —$CONH_2$ or —CONH($C_1$—$C_4$ alkyl), most preferably —$CONH_2$, —$CONH.CH_3$ or —$CONH.H_5$.

$R^5$ and $R^6$ are both preferably H.

In the most preferred individual compounds, R is 2,4-dichlorophenyl; $R^1$ is —$CONH_2$, —$CONHCH_3$ or —$CONHC_2H_5$; and $R^5$ and $R^6$ are H.

In one aspect of the invention, there are provided compounds of the formula:—

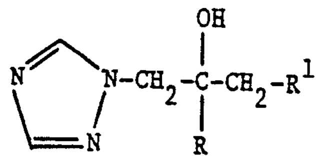

where

R is phenyl optionally substituted by 1 to 3 substituents each independently selected from F, Cl, Br, I, $CF_3$, $C_1$—$C_4$ alkyl and $C_1$—$C_4$ alkoxy, or R is a 5-chloropyrid-2-yl group; and

$R^1$ is —CN or —$CON^2R^3$ where $R^2$ is H, or $C_1$—$C_6$ alkyl, and $R^3$ is H, $C_1$—$C_6$ alkyl, benzyl, —$CH_2CF_3$ or adamantyl, said benzyl group being optionally substituted on its phenyl ring portion by 1 or 2 substituents each independently selected from $C_1$—$C_4$ alkyl, $C_1$—$C_4$ alkoxy, F, Cl, Br, I and $CF_3$, or $R^2$ and $R^3$ taken together with the nitrogen atom to which they are attached represent a group of the formula

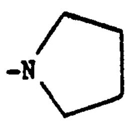 , 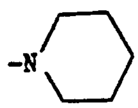 , 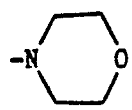 or 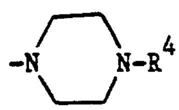

where

$R^4$ is H, $C_1$—$C_4$ alkyl, $C_2$—$C_4$ alkanoyl or ($C_1$—$C_4$ alkoxy)carbonyl;

and their pharmaceutically acceptable salts.

In another aspect of the invention, there are provided compounds of the formula:—

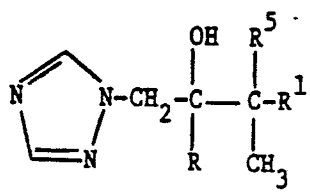

where

R is phenyl optionally substituted by 1 to 3 substituants each independently selected from F, Cl, Br, I, $CF_3$, $C_1$—$C_4$ alkyl and $C_1$—$C_4$ alkoxy, or R is a 5-chloropyrid-2-yl group; and

$R^1$ is —CN or —$CONR^2R^3$ where $R^2$ is H or $C_1$—$C_6$ alkyl, and $R^3$ is H, $C_1$—$C_6$ alkyl, benzyl, —$CH_2CF_3$ or adamantyl, said benzyl group being optionally substituted on its phenyl ring portion by 1 to 2 substituents each independently selected from $C_1$—$C_4$ alkyl, $C_1$—$C_4$ alkoxy, F, Cl, Br, I and $CF_3$, or $R^2$ and $R^3$ taken together with the nitrogen atom to which they are attached represent a group of the formula

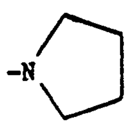 , 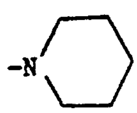 , 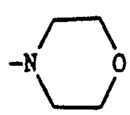 or 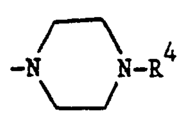

where

$R^4$ is H, $C_1$—$C_4$ alkyl, $C_2$—$C_4$ alkanoyl or ($C_1$—$C_4$ alkoxy)carbonyl; and

$R^5$ is H or $CH_3$;

and their pharmaceutically acceptable salts.

In a further aspect of the invention, there are provided compounds of the formula:—

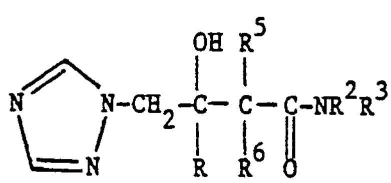

where

R is a phenyl group optionally substituted by 1 to 3 substituents each independently selected from F, Cl, Br, I, $CF_3$, $C_1$—$C_4$ alkyl and $C_1$—$C_4$ alkoxy, or R is a 5-chloropyrid-2-yl group;

$R^5$ and $R^6$ are each independently H or $CH_3$;

and either

(a) $R^2$ is H or $C_1$—$C_4$ alkyl and $R^3$ is $C_2$—$C_4$ alkanoyl or benzoyl, said benzoyl groups being optionally substituted by 1 or 2 substituents each independently selected from F, Cl, Br, I, $CF^3$, $C_1$—$C_4$ alkyl and $C_1$—$C_4$ alkoxy; or

(b) $R^3$ and $R^4$ are both ($C_1$—$C_4$ alkoxy)carbonyl; and their pharmaceutically acceptable salts.

Where the compounds of formula (I) contain at least one chiral centre, the invention includes both the resolved and unresolved forms.

Some useful intermediates have the formula:—

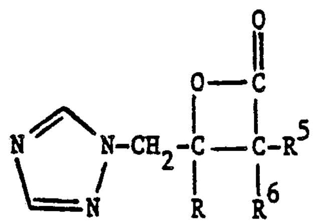

--- (A)

where R, $R^5$ and $R^6$ are as previously defined for formula (I).

Other useful intermediates have the formula:—

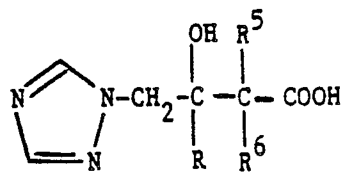

--- (B)

where R is as defined for formula (I).

The compounds of the formula (I) can be prepared as follows:—

(1) Compounds in which $R^1$ is —CN and $R^5$ and $R^6$ are H can be prepared by the following general method:—

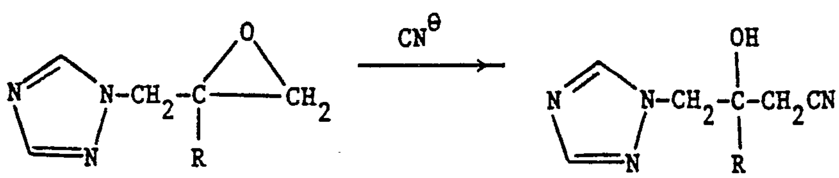


(II)

The preferred source of cyanide ions are the alkali metal cyanides, particularly sodium and potassium cyanide. In a typical procedure, the compound (II) and sodium or potassium cyanide are heated together in a suitable organic solvent, e.g. dimethylformamide, at up to 100°C, preferably 65—70°C, for, say, up to 6 hours. It is preferred to add the cyanide dropwise to the solution of the oxirane over, say, ½ hour. After cooling the reaction mixture and pouring it into water, the desired product can be isolated and purified by conventional techniques.

The starting materials of the formula (II) are in many cases known compounds (see e.g. European Patent Application Publication No. 44605 to I.C.I.) or can be prepared by routine methods as will be known to those skilled in the art, e.g.:—

(a)

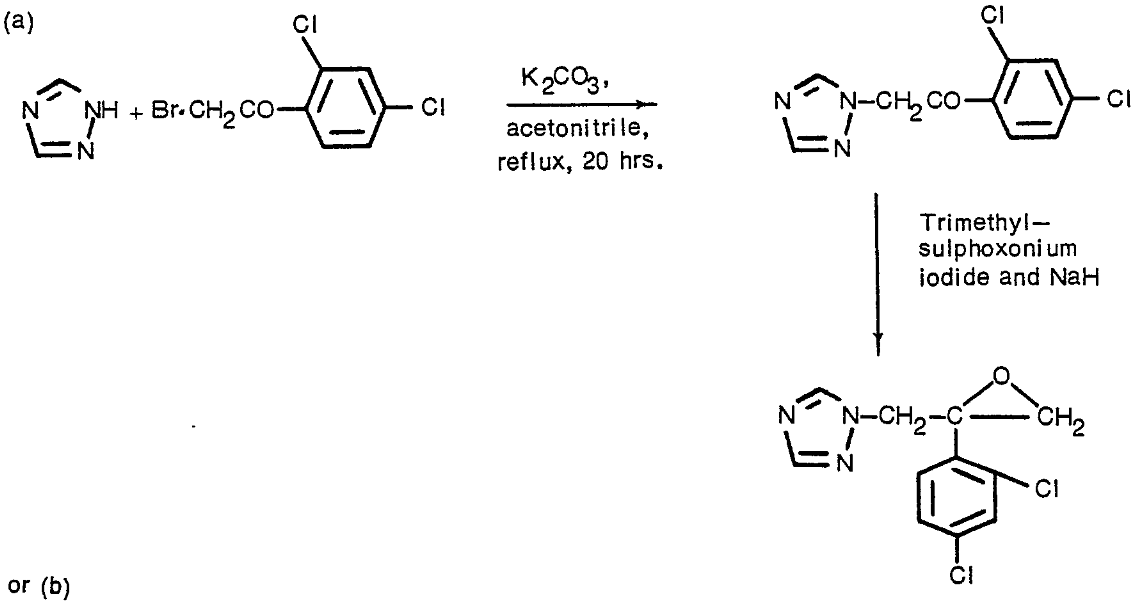


or (b)

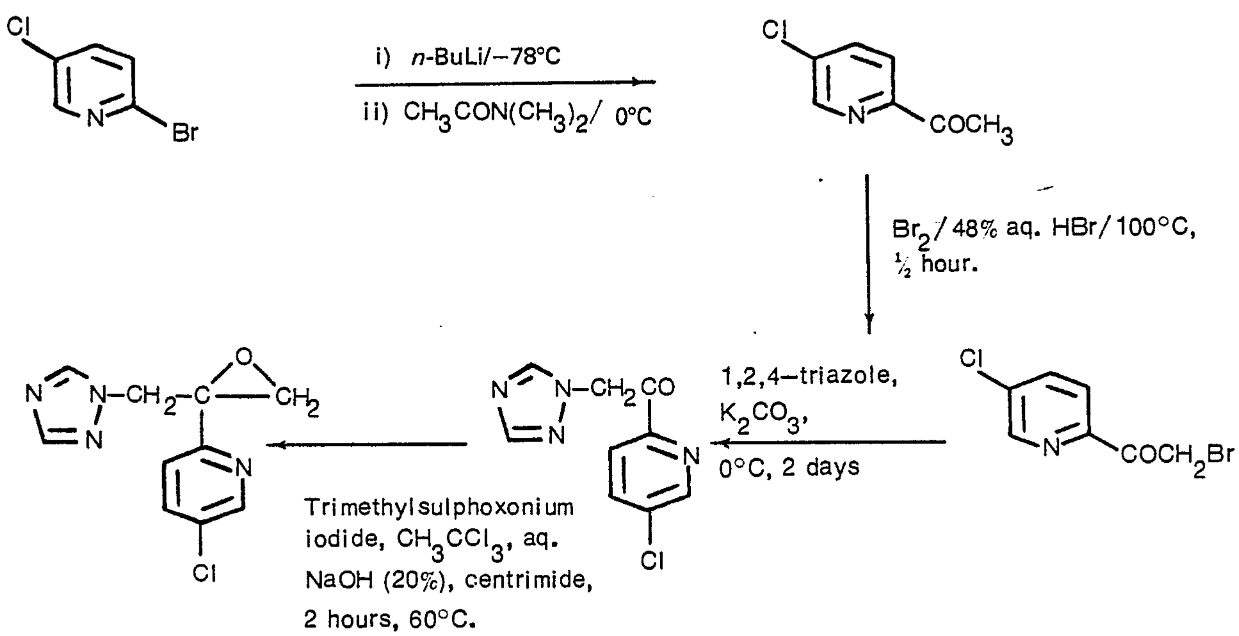


(2) Compounds in which $R^1$ is —CN and $R^5$ and $R^6$ are each H or $CH_3$ can be prepared by the following general route:—

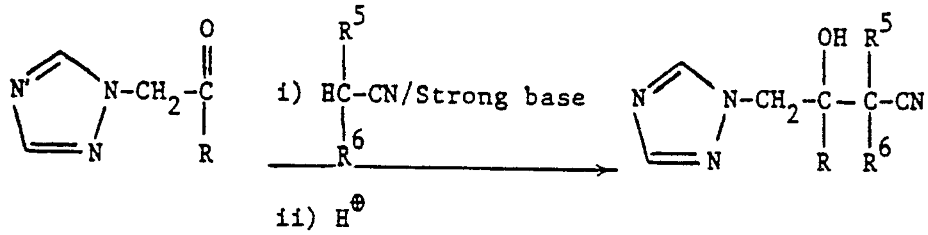


(III)

The preferred strong base in *n*-butyllithium. In a typical procedure, the nitrile is dissolved in a suitable solvent, e.g. dry tetrahydrofuran (THF), and the resulting solution is then cooled to about −70°C. A solution of *n*-butyllithium in hexane is then slowly added dropwise. After stirring for about 1 hour at −70°C, the ketone (III) in a suitsble solvent, e.g. dry THF, is slowly added dropwise. After stirring for about an hour at −70°C glacial acetic acid in a little THF is added and the reaction mixture is allowed to warm to 0°C. The

product can then be isolated and purified conventionally. When one of $R^5$ and $R^6$ is H and the other is $CH_3$, the product will exist in two diastereoisomeric forms and these can often be separated by chromatography (see Example 34).

The starting materials of the formula (III) are either known compounds or can be prepared by conventional methods (see e.g. Preparations 3(i) and 4(B)).

(3) Compounds in which $R^1$ is $—CONH_2$ and $R^5$ and $R^6$ are H can be prepared as follows:—

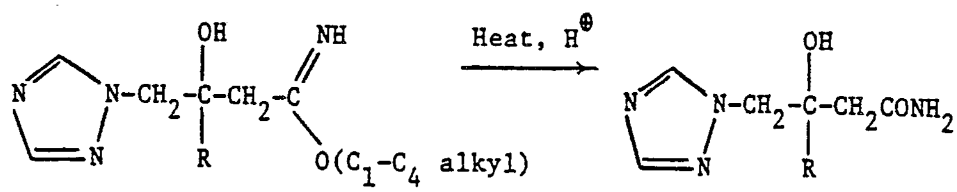


(IV)

Preferably compound (IV) is used in its ethyl ester form. The acid is preferably supplied by using compound (IV) in an acid addition salt form, e.g. as the dihydrochloride. Alternatively the free base can be used and hydrogen chloride gas can be bubbled into the solution. Typically the reactants are heated together for a short period, preferably under reflux in a suitable high-boiling organic solvent such as 1,2-dichlorobenzene (b.p. 178°C), when the reaction is usually complete in about 15 minutes.

The starting materials of the formula (IV) are obtainable conventionally, e.g. as follows:—

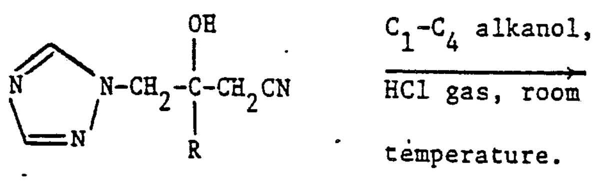


(IV) (as dihydro-chloride salt)

(4) Compounds in which $R^1$ is $—CONH(C_1—C_4$ alkyl) or $—CON(C_1—C_4$ alkyl$)_2$ can be prepared by the alkylation of the corresponding starting materials in which $R^1$ is $—CONH_2$. The alkylation is typically carried out by dissolving the starting material in a suitable organic solvent, e.g. dry THF, followed by cooling to 0—5°C. A strong base such as sodium hydride is then added. After stirring for a few minutes, an appropriate quantity of alkylating agent is added. The preferred alkylating agents are the alkali metal iodides and bromides. For mono-alkylation, only one equivalent of alkylating agent should be used, and, for dialkylation, at least 2 equivalents. The alkylated product can be isolated from the reaction mixture by conventional techniques.

(5) Compounds in which $R^1$ is $—CONR^2R^3$ where $R^2$ and $R^3$ are as defined in (a) or (b) in formula (I) can also be prepared as follows:—

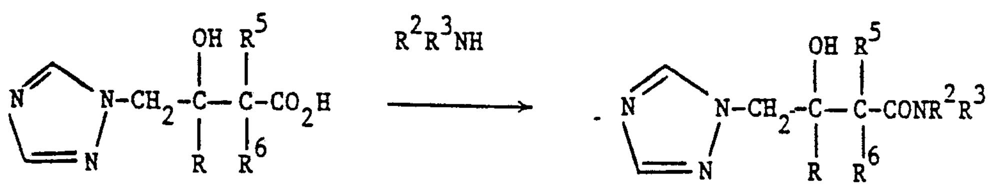


(V)

Compound (V) is preferably used in the form of its "functional equivalent as an acylating agent", e.g. as an acid chloride or bromide, a mixed anhydride of the formula:—

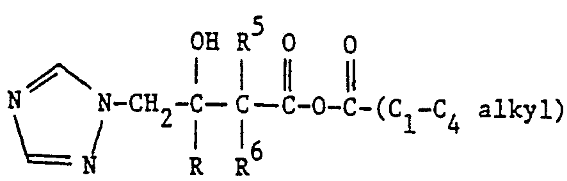

--- (VI),

or as a $C_1—C_4$ alkyl, succinimido, phthalimido or benzotriazol-1-yl ester.

All these "functional equivalents" are preparable conventionally from the acid (V). The acid chlorides

and bromides are for example preparable by reaction of said acid with thionyl chloride or bromide, the mixed anhydrides by reaction with a $C_2$—$C_5$ alkanoyl chloride, the $C_1$—$C_4$ alkyl esters by simple esterification, and the succinimido, phthalimido and benzotriazol-1-yl esters by reaction with N-hydroxysuccinimide, N-hydroxyphthalimide or 1-hydroxybenzotriazole in the presence of a dehydrating agent such as dicyclohexylcarbodiimide.

In fact, it is preferred to use the compounds (V) in the form of their succinimido esters of the formula:—

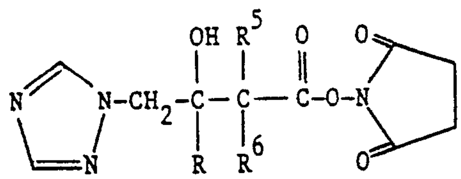

--- (VII).

Thus in a typical procedure, dicyclohexylcarbodiimide dissolved in e.g. dry dioxan is added to a solution of the acid (V) and N-hydroxysuccinimide in e.g. dry dioxan. After stirring for a few hours at room temperature and filtering, the reaction is generally completed by stirring the solution of the compound (VII) with the amine $R^2R^3NH$ at room temperature for a few hours in e.g. dry dioxan, after which the product can be isolated and purified by conventional means.

If compound (V) is reacted in its free acid form, the reaction should generally be carried out in the presence of a dehydrating agent such as dicyclohexylcarbodiimide.

The $C_1$—$C_4$ alkyl esters can also be prepared as follows:—

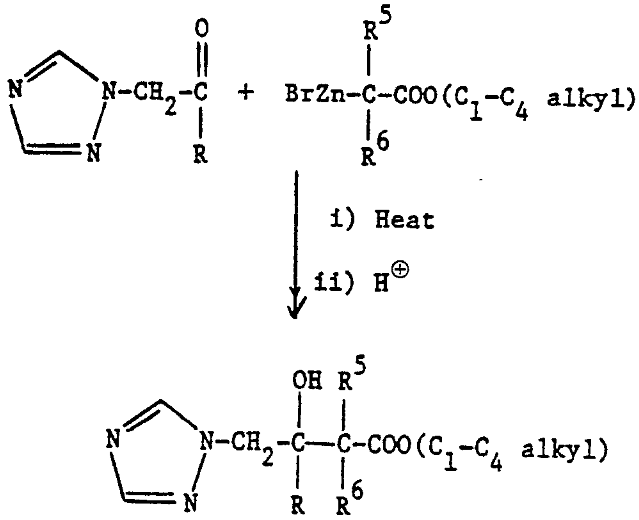


--- (VIII)

Generally some of the product (VIII) cyclises *in situ* under the reaction conditions to give the intermediate lactone (A). The ester (VIII) and lactone (A) can be separated chromatographically.

The benzotriazol-1-yl esters have the formula:—

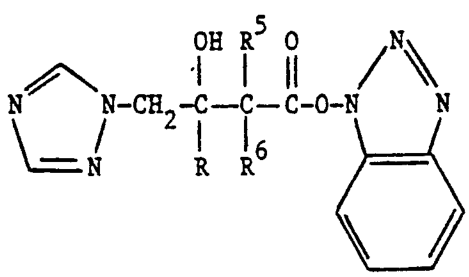

--- (IX).

These can be prepared as stated above.

Thus in a typical procedure, dicyclohexylcarbodiimide, 1-hydroxybenzotriazole and the acid (V) are stirred together at room temperature for a short period in e.g. dry dioxan. The reaction is generally completed by stirring the resulting intermediate (IX) with the amine $R^2R^3NH$ at room temperature until the reaction is complete, after which the product can be isolated and purified by conventional means.

(6) Compounds of the formula (I) in which $R^1$ is —$CONH_2$ can be prepared by the controlled hydrolysis

of the corresponding compounds in which $R^1$ is —CN. Typically this hydrolysis is carried out by heating the starting nitrile at, say, 70—100°C, preferably 90—95°C, with aqueous sulphuric acid, preferably 80%, until the formation of the amide is complete as monitored by t.l.c. Further hydrolysis to convert $-CONH_2$ to —COOH can be carried out under similar conditions, if desired. The compounds in which $R^1$ is —COOH are useful intermediates [see route (5) above.

(7) The amides of the formula (I) in which $R^1$ is $-CONR^2R^3$ where $R^2$ and $R^3$ are as defined in (a) or (b) for formula (I) can also be prepared from the intermediates of the formula (A) as follows:—

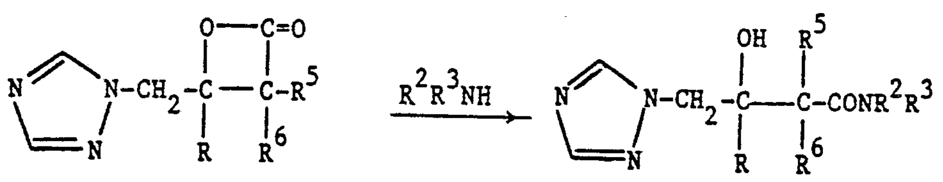

(A)

where R, $R^5$ and $R^6$ are as defined for formula (I), and $R^2$ and $R^3$ are as defined in (a) or (b) in formula (I).

The reaction can be carried out by stirring the reactants together in a suitable solvent, e.g. ethanol, at room temperature until the reaction is complete. If necessary, the reaction mixture can be heated to accelerate the reaction. The product can be isolated and purified conventionally.

(8) The compounds of the formula (I) in which $R^2$ is H or $C_1$—$C_4$ alkyl and $R^3$ is $C_2$—$C_4$ alkanoyl or said optionally substituted benzoyl group, can be prepared by reacting a compound of the formula:—

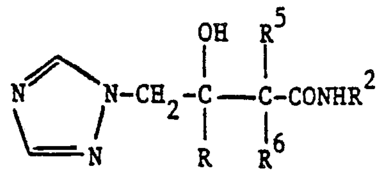
--- (X)

where $R^2$ is as defined above in this method and R, $R^5$ and $R^6$ are as defined for formula (I), with either an acid halide or an acid anhydride of the formula:—

$R^3.X$ or $(R^3)_2O$

where $R^3$ is as defined above in this method and X is Cl or Br.

When an acid halide is used, the reaction is desirably carried out in the presence of a base such as pyridine or sodium hydride.

The reaction is typically carried out in a suitable organic solvent, e.g. acetonitrile or tetrahydrofuran. It is not generally necessary to accelerate the reaction by heating, and the product can be isolated and purified conventionally.

(9) The compounds of formula (I) in which $R^2$ and $R^3$ are both ($C_1$—$C_4$ alkoxy)carbonyl can be prepared by reacting a compound of the formula:—

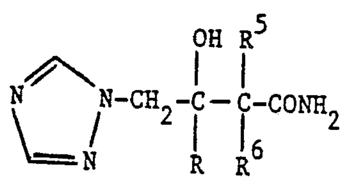
--- (XI)

with at least two equivalents of an alkyl haloformate of the formula:

$$X-\underset{\underset{O}{\|}}{C}-O-(C_1-C_4\ \text{alkyl})$$

where X is Cl or Br.

The reaction can be carried out in a similar manner to route (8) above and again the presence of a base is desirable.

(10) Compounds in which $R^3$ is 2-(methylsulphinyl)ethyl and 2-(methylsulphonyl)ethyl can be prepared by the oxidation of the corresponding 2-(methylthio)ethyl compounds using the appropriate quantity of *m*-chloroperbenzoic acid in conventional manner.

(11) Compounds in which $R^1$ is $—CSNH_2$ can be prepared as follows:—

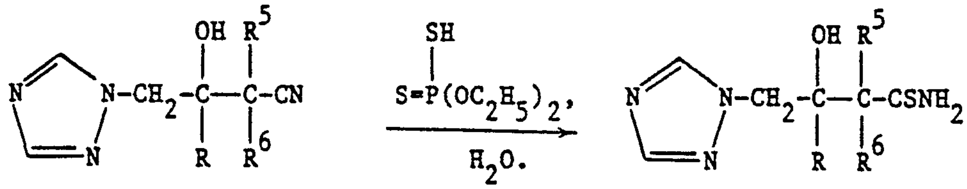

The reaction is typically carried out by heating the reactants at up to about 100°C in the presence of water. The product can then be isolated conventionally.

(12) Compounds in which $R^1$ is $—CONH_2$ and $R^5$ and $R^6$ are H can also be prepared as follows:—

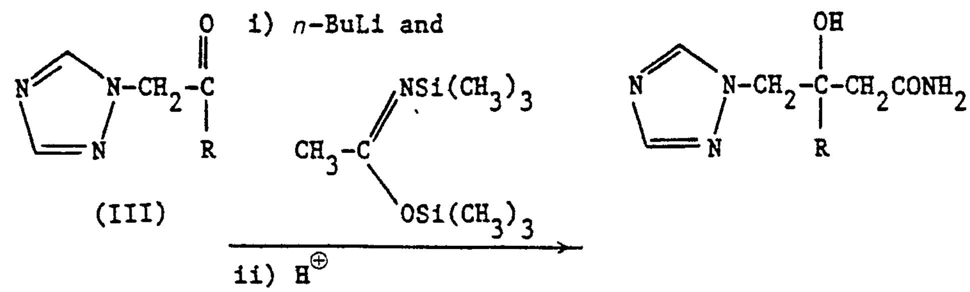


(III)

The reaction is typically carried out by stirring bis(trimethylsilyl)acetamide at −70°C in dry tetrahydrofuran (THF) whilst *n*-butyllithium is slowly added dropwise. The resulting solution is stirred at about −70°C for a short period, then the ketone (III) in e.g. dry THF is slowly added, and the resulting mixture stirred at about −70°C for a few hours. The reaction mixture is then allowed to warm to room temperature and aqueous acid is added. The product can then be isolated and purified conventionally.

(13) Compounds in which $R^2$ and $R^3$ together with the nitrogen atom to which they are attached represent aziridinyl can be prepared as follows:—

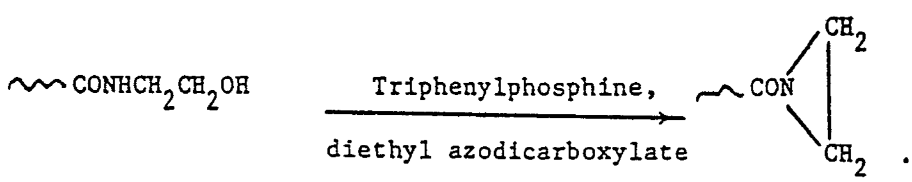


The reaction is generally carried out at room temperature in dry tetrahydrofuran.

(14) The lactone intermediates of the formula (A) can be prepared by cyclisation, preferably using an ester according to the following scheme:—

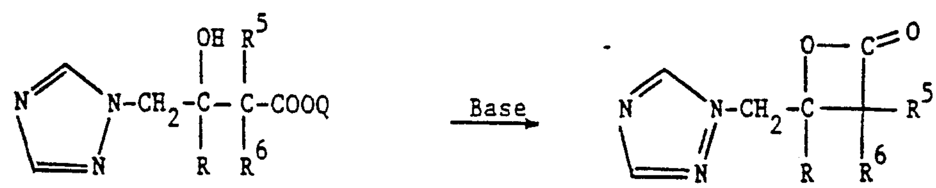


(XII) (A)

where Q = $C_1$—$C_4$ alkyl, phthalimido, succinimido or 1-benzotriazolyl.

These esters can be prepared as previously described. The cyclisation can be carried out in the presence of a suitable base by stirring at room temperature. Preferred bases are tertiary amine bases, e.g. triethylamine, and alkali metal hydrides, e.g. sodium hydride.

The compounds of the invention contain a chiral centre or centres and the invention includes both the resolved and unresolved forms.

Pharmaceutically acceptable acid addition salts of the compounds of the formula (I) are those formed from strong acids which form non-toxic acid addition salts, such as hydrochloric, hydrobromic, sulphuric,

oxalic and methanesulphonic acids.

The salts may be obtained by conventional procedures, e.g. by mixing solutions containing equimolar amounts of the free base and desired acid, and the required salt is collected by filtration, if insoluble, or by evaporation of the solvent.

Also included are the alkali metal salts, preparable conventionally.

The compounds of the formula (I) and their pharmaceutically acceptable salts are antifungal agents, useful in combating fungal infections in animals, including humans. For example they are useful in treating topical fungal infections in man caused by, among other organisms, species of *Candida, Trichophyton, Microsporum* or *Epidermophyton*, or in mucosal infections caused by *Candida albicans* (e.g. thrush and vaginal candidiasis). They can also be used in the treatment of systemic fungal infections caused by, for example, *Candida albicans, Cryptococcus neoformans, Aspergillus fumigatus, Coccidioides, Paracoccidioides, Histoplasma* or *Blastomyces*.

The *in vitro* evaluation of the antifungal activity of the compounds can be performed by determining the minimum inhibitory concentration (m.i.c.) which is the concentration of the test compounds in a suitable medium at which growth of the particular micro-organism fails to occur. In practice, a series of agar plates, each having the test compound incorporated at a particular concentration is inoculated with a standard culture of, for example, *Candida albicans* and each plate is then incubated for 48 hours at 37°C. The plates are then examined for the presence or absence of growth of the fungus and the appropriate m.i.c. value is noted. Other micro-organisms used in such tests can include *Cryptococcus neoformans, Aspergillus fumigatus, Trichophyton* spp; *Microsporum* spp; *Epidermophyton floccosum, Coccidioides immitis* and *Torulopsis glabrata*.

The *in vivo* evaluation of the compounds can be carried out at a series of dose levels by intraperitoneal or intravenous injection or by oral administration, to mice which are inoculated with a strain of *Candida albicans*. Activity is based on the survival of a treated group of mice after the death of an untreated group of mice following 48 hours observation. The dose level at which the compound provides 50% protection ($PD_{50}$) against the lethal effect of the infection is noted.

For human use, the antifungal compounds of the formula (I) can be administered alone, but will generally be administered in admixture with a pharmaceutical carrier selected with regard to the intended route of administration and standard pharmaceutical practice. For example, they can be administered orally in the form of tablets containing such excipients as starch or lactose, or in capsules or ovules either alone or in admixture with excipients, or in the form of elixirs or suspensions containing flavouring or colouring agents. They can be injected parenterally, for example, intravenously, intramuscularly or subcutaneously. For parenteral administration, they are best used in the form of a sterile aqueous solution which may contain other substances, for example, enough salts or glucose to make the solution isotonic with blood.

For oral and parenteral administration to human patients, the daily dosage level of the antifungal compounds of the formula (I) will be from 0.1 to 5 mg/kg (in dividend doses) when administered by either the oral or parenteral route. Thus tablets or capsules of the compounds will contain from 5 mg to 0.5 g of active compound for administration singly or two or more at a time as appropriate. The physician in any event will determine the actual dosage which will be most suitable for an individual patient and it will vary with the age, weight and response of the particular patient. The above dosages are exemplary of the average case; there can, of course, be individual instances where higher or lower dosage ranges are merited, and such are within the scope of this invention.

Alternatively, the antifungal compounds of formula (I) can be administered in the form of a suppository or pessary, or they may be applied topically in the form of a lotion, solution, cream, ointment or dusting powder. For example, they can be incorporated into a cream consisting of an aqueous emulsion of polyethylene glycols or liquid paraffin; or they can be incorporated, at a concentration between 1 and 10%, into an ointment consisting of a white wax or white soft paraffin base together with such stabilizers and preservatives as may be required.

The following Examples illustrate the invention. All temperatures are in °C:—

## Example 1

Preparation of 1-cyano-2-(2,4-dichlorophenyl)-3-(1H-1,2,4-triazol-1-yl)propan-2-ol

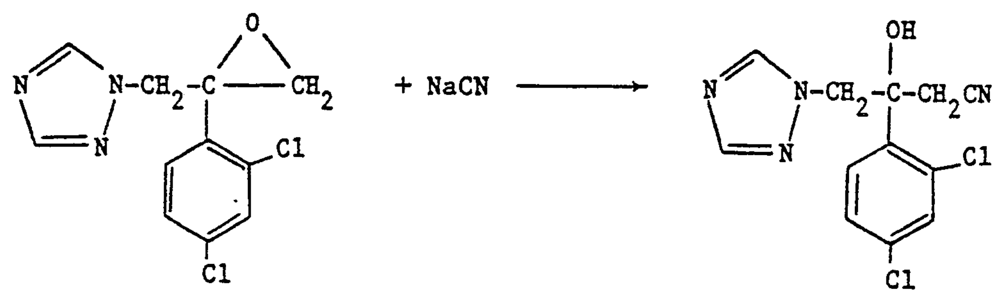


To 2-(2,4-Dichlorophenyl)-2-(1H-1,2,4-triazol-1-methyl)oxirane (6.7 g) is dimethylformamide (198 ml) at

60° was added sodium cyanide (2.84 g) in water (49 ml) dropwise over 25 minutes. Heating at 60° was continued for 5 hours. The reaction mixture was then cooled, poured into water (900 ml), and extracted with ethyl acetate (3 × 150 ml). The combined organic extracts were washed with saturated aqueous brine, dried ($Na_2SO_4$) and evaporated to dryness to give a pale yellow solid (6.1 g) which was triturated with ether. The residual solid was recrystallised from ether/methanol to give the title compound, 4.13 g (56%), m.p. 217—219°.

*Analysis %:—*

| | | | |
|---|---|---|---|
| Found: | C, 48.3; | H, 3.4; | N, 18.4 |
| Calculated for $C_{12}H_{10}Cl_2N_4O$: | C, 48.5; | H, 3.4; | N, 18.8. |

Example 2

Preparation of 1-cyano-2-(2,4-dichlorophenyl)-3-(1H-1,2,4-triazol-1-yl)propan-2-ol

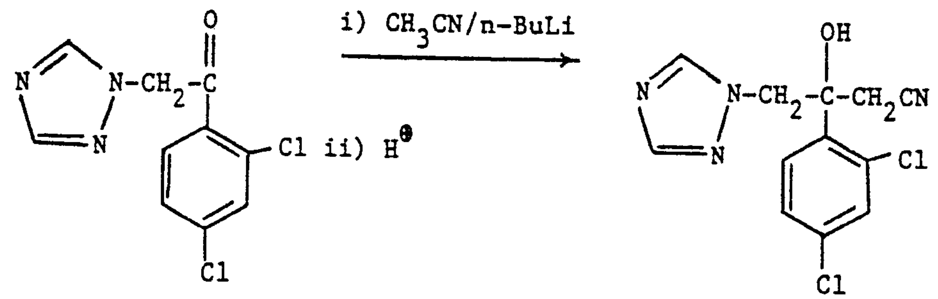


Acetonitrile (2.25 g, 1.1 equivalent) was dissolved in dry tetrahydrofuran (100 ml) and the resulting solution was cooled to −70° under nitrogen in a $CO_2$/acetone bath. A solution of *n*-butyllithium in hexane (39 ml, 1.55 molar, 1.2 equivalent) was added dropwise over a 5 minute period. After stirring for about 45 minutes at −70°, 2′,4′-dichloro-2-(1H-1,2,4-triazol-1-yl) acetophenone (12.8 g) in dry tetrahydrofuran (100 ml) was added dropwise over a 15 minute period. Stirring was continued at −70° for about 1 hour and then glacial acetic acid (20 ml) in tetrahydrofuran (20 ml) was added dropwise. The cooling bath was then removed. The reaction mixture was allowed to warm to 0°C, quenched in water (400 ml), and solid sodium carbonate was added to raise the pH to 8.0. After extraction with ethyl acetate (3 × 75 ml), the combined organic extracts were washed with saturated brine (3 × 50 ml), dried ($Na_2SO_4$) and evaporated to a pale yellow solid. This solid was washed well with ether to give the title compound (6.61 g, 44.5%), identical to the product of Example 1 as confirmed by n.m.r. and i.r. spectroscopy.

Example 3

1-Cyano-2-(2,4-difluorophenyl)-3-(1H-1,2,4-triazol-1-yl)propan-2-ol was prepared similarly to the previous Example using 2′,4′-difluoro-2-(1H-1,2,4-triazol-1-yl)acetophenone as the starting ketone. It had an m.p. of 154—5°.

*Analysis %:—*

| | | | |
|---|---|---|---|
| Found: | C, 54.0; | H, 3.8; | N, 21.5 |
| Calculated for $C_{12}H_{10}F_2N_4O$: | C, 54.6; | H, 3.8; | N, 21.2. |

Example 4

Preparation of 1-carbamoyl-2-(2,4-dichlorophenyl)-3-(1H-1,2,4-triazol-1-yl)propan-2-ol

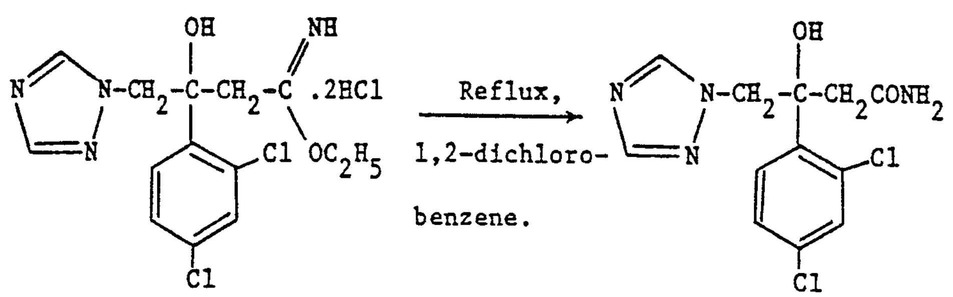


3-(2,4-Dichlorophenyl)-3-hydroxy-4-(1H-1,2,4-triazol-1-yl) butyrimidic acid, ethyl ester dihydrochloride (3.42 g) was suspended in 1,2-dichlorobenzene (35 ml) and the reaction mixture was heated to the reflux temperature of the solvent (178°). After refluxing for 5 minutes, a solution was obtained. Refluxing was then continued for a further 10 minutes. The reaction mixture was then cooled, evaporated, and the resulting gum was triturated with hexane and heated with acetone. On cooling a cream coloured granular solid was

formed which was filtered off to yield the title compound as a solvate (1.26 g), On standing overnight in a refrigerator some further solvated product precipitated (0.62 g). After drying at 80°C for 6 hours to remove the solvent the pure title compound was obtained, yield 1.5 g, m.p. 144—145°.

*Analysis* % (after said drying):—

| | | | |
|---|---|---|---|
| Found: | C, 45.5; | H, 3.8; | N, 17.5 |
| Calculated for $C_{12}H_{12}Cl_2N_4O_2$: | C, 45.7; | H, 3.8; | N, 17.8. |

Example 5

Preparation of 2-(2,4-dichlorophenyl)-1-(N-methylcarbamoyl)-3-(1H-1,2,4-triazol-1-yl)propan-2-ol

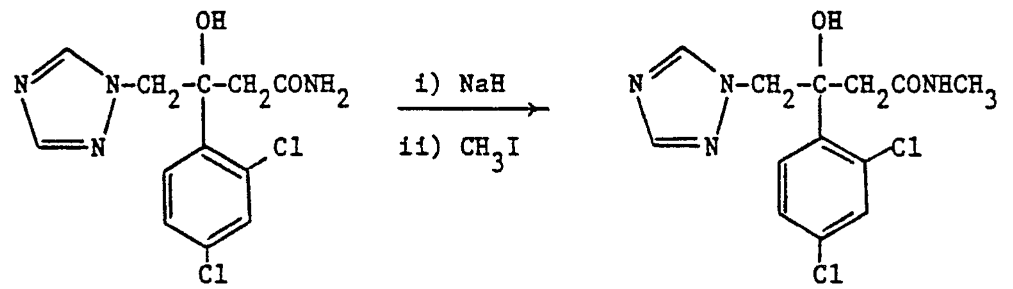


1-Carbamoyl-2-(2,4-dichlorophenyl)-3-(1H-1,2,4-triazol-1-yl)propan-2-ol (1.0 g) was dissolved in dry tetrahydrofuran (20 ml) and the reaction mixture was cooled to 0—5°. Sodium hydride (0.15 g, as a 50% dispersion in oil) was then added, followed by, after stirring for 10 minutes, methyl iodide (0.45 g). Further quantities of methyl iodide (90 mg) and sodium hydride (375 mg, as a 50% dispersion in oil) were added. After stirring for a few minutes, yet further quantities of methyl iodide (90 mg) and sodium hydride (375 mg, as a 50% dispersion in oil) were added. The mixture was then quenched in water and extracted with ethyl acetate (3 × 50 ml). The combined organic extracts were dried ($MgSO_4$) and evaporated to give the crude product as a gum. A solution of this gum in methylene chloride (20 ml) was chromatographed on a "Kieselgel 60H" (Trade Mark) column (10 g), eluting with methylene chloride (100 ml), then with methylene chloride containing 2 vol.% isopropanol and 0.2 vol.% $NH_4OH$ (300 ml), and finally with methylene chloride containing 5 vol.%, isopropanol and 0.5 vol.% $NH_4OH$ (500 ml). Appropriate fractions were collected to yield the title compound, which was recrystallised from cyclohexane (yield 41 mg, m.p. 151—4°).

*Analysis %*:—

| | | | |
|---|---|---|---|
| Found: | C, 47.3; | H, 4.35; | N, 17.2 |
| Calculated for $C_{13}H_{14}Cl_2N_4O_2$: | C, 47.4; | H, 4.30; | N, 17.0. |

Example 6

Preparation of 2-(2,4-dichlorophenyl)-1-(morpholinocarbonyl)-3-(1H-1,2,4-triazol-1-yl)propan-2-ol monohydrate

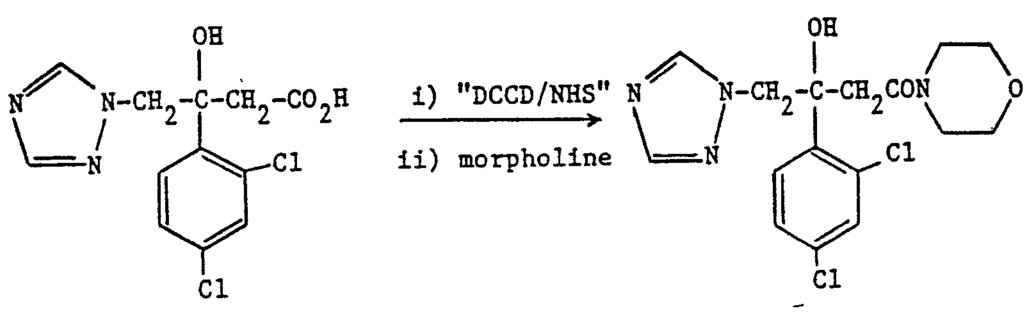


N,N'-Dicyclohexylcarbodiimide ("DCCD") (110 mg, 0.5 mmole) dissolved in dry dioxan (5 ml) was added to a solution of 1-carboxy-2-(2,4-dichlorophenyl)-3-(1H-1,2,4-triazol-1-yl)propan-2-ol (150 mg, 0.5 mmole) and N-hydroxysuccinimide ("NHS") (60 mg, 0.5 mmole) in dry dioxan (10 ml), and the mixture was stirred at room temperature for 2 hours. The precipitate was filtered off, washed with dry dioxan (10 ml) and the combined filtrate and washings were then added to a solution of morpholine (300 mg, 3.4 mmole) in dry dioxan (2 ml). The resultant solution was left at room temperature for 18 hours, diluted with ethyl acetate (100 ml), washed three times with saturated brine solution and dried over magnesium sulphate. Evaporation of the filtrate gave an oil (300 mg) which was then chromatographed on "Kieselgel 60H" (Merck, Trade Mark) silica (10 g), eluting with $CH_2Cl_2$ containing 2 vol.% isopropyl alcohol and 0.2 vol.% aq. ammonium hydroxide (S.G. 0.880). The title compound was obtained after evaporation of appropriate fractions as a colourless solid, 110 mg (57%), m.p. 92—93°.

*Analysis %:—*
Found: C, 47.8; H, 4.7; N, 13.9
Calculated for $C_{16}H_{18}N_4Cl_2O_3.H_2O$: C, 47.8; H, 5.0; N, 13.9.

Examples 7—32

The following compounds were prepared similarly to the previous Example, starting from the same acid, DCCD/NHS and the appropriate amine:—

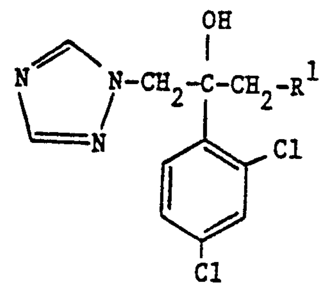

| Example No. | $R^1$ | m.p. (°C) | Analysis % Theoretical in brackets C | H | N |
|---|---|---|---|---|---|
| 7 | $-CONHCH_3$ | 151-3° | (Identical to the product of Example 5) | | |
| 8 | $-CONHCH(CH_3)_2$ | 105-107° | 50.7 (50.6 | 5.2 5.1 | 15.3 15.7) |
| 9 | $-CON(CH_3)_2$ | 125-126.5° | 48.95 (49.1 | 4.65 4.7 | 16.3 16.4) |
| 10 | $-CONHCH_2-$ 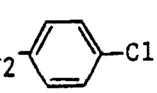 $-Cl$ | glass 64-65° | 52.15 (52.05 | 3.95 3.9 | 12.5 12.8) |

| Example No. | $R^1$ | m.p. (°C) | Analysis % Theoretical in brackets C | H | N |
|---|---|---|---|---|---|
| 11 | 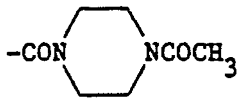 | glass, 63-5° | (As trihydrate) 45.3 | 4.4 | 14.4 |
| | | | (45.1 | 5.6 | 14.6) |
| 12 | 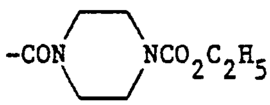 | glass, 58-60° | (As hemihydrate) 49.2 | 5.1 | 15.0 |
| | | | (49.1 | 5.2 | 15.1) |
| 13 | 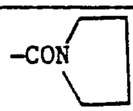 | glass, | 52.4 | 5.05 | 15.0 |
| | | 40-41° | (52.2 | 4.9 | 15.2) |
| 14 | $-CON(C_2H_5)_2$ | glass, | 51.9 | 5.5 | 15.0 |
| | | 60-62° | (51.8 | 5.4 | 15.1) |
| 15 | $-CONHC_2H_5$ | 129-130° | 49.0 | 4.8 | 15.8 |
| | | | (49.0 | 4.7 | 16.3) |
| 16 | -CONH(1-adamantyl) | 91-92° | 58.7 | 6.1 | 12.0 |
| | | | (58.8 | 5.8 | 12.5) |
| 17 | 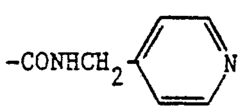 | glass, 48-50° | (as hemihydrate) 52.3 | 4.2 | 16.5 |
| | | | (52.1 | 4.3 | 16.8) |
| 18 | $-CONHCH_2CF_3$ | glass, 60-62° | 42.6 | 3.5 | 13.6 |
| | | | (42.3 | 3.3 | 14.1) |
| 19 | $-CONH(CH_2)_5CH_3$ | 114-116° | 54.2 | 6.1 | 14.1 |
| | | | (54.1 | 6.1 | 14.0) |

| Example No. | $R^1$ | m.p. (°C) | Analysis % Theoretical in brackets C | H | N |
|---|---|---|---|---|---|
| 20 | 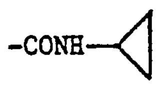 | 122-123° | 50.6<br>(50.7 | 4.6<br>4.5 | 15.6<br>15.8) |
| 21 | 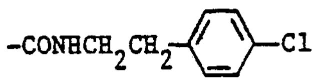 | 142-143° | 52.9<br>(52.9 | 4.2<br>4.2 | 12.2<br>12.3) |
| 22 | $-CONH(CH_2)_2CH_3$ | 169-170° | (As hydrochloride)<br>45.7<br>(45.8 | <br>4.8<br>4.9 | <br>14.0<br>14.2) |
| 23 | $-CONHCH_2CONH_2$ | 188-191° | (As hydrochloride hemihydrate)<br>40.6<br>(40.2 | <br>4.05<br>4.1 | <br>16.9<br>16.8) |
| 24 | $-CONHCH_2-CH=CH_2$ | glass | (As oxalate ½ hydrate)<br>45.4<br>(45.4 | <br>4.3<br>4.1 | <br>12.0<br>12.4) |
| 25 | $-CONHCH_2C(CH_3)_3$ | 135-137° | 53.2<br>(53.0 | 5.9<br>5.8 | 14.7<br>14.5) |
| 26 | $-CONHCH_2CH_2OH$ | 143-145° | (As 1/4 hydrate)<br>46.1<br>(46.2 | <br>4.4<br>4.6 | <br>15.3<br>15.4) |
| 27 | 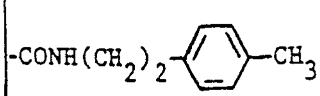 | 144-145° | 58.2<br>(58.2 | 5.1<br>5.1 | 12.8<br>12.9) |

| Example No. | $R^1$ | m.p. (°C) | Analysis % Theoretical in brackets C | H | N |
|---|---|---|---|---|---|
| 28 | $-CONH(CH_2)_2N(CH_3)_2$ | 107–110° | (As dihydrochloride dihydrate) 38.8 | 5.2 | 13.6 |
| | | | (38.8 | 5.1 | 14.2) |
| 29 | 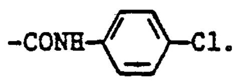 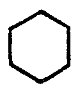 | 102–105° | (Contains 1 mole of cyclohexane ) 56.6 | 5.4 | 10.9 |
| | | | (56.5 | 5.3 | 11.0) |
| 30 | $-CONHCH_2CH_2SCH_3$ | 154–156° | 42.6 | 4.5 | 13.3 |
| | | | (42.3 | 4.5 | 13.2) |
| 31 | 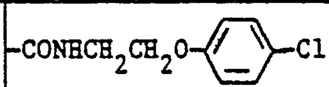 | 137–9° | 51.0 | 4.0 | 11.9 |
| | | | (51.1 | 4.1 | 11.9) |
| 32 | $-CON(CH_3)(CH[CH_3]_2)$ | 131–2° | 51.7 | 5.3 | 15.2 |
| | | | (51.8 | 5.4 | 15.1) |

Example 33

The following compound was prepared similarly to Example 6 starting from 1-carboxy-2-(2,4-difluorophenyl)-3-(1H-1,2,4-triazol-1-yl)propan-2-ol, "DCCD", "NHS" and methylamine:—

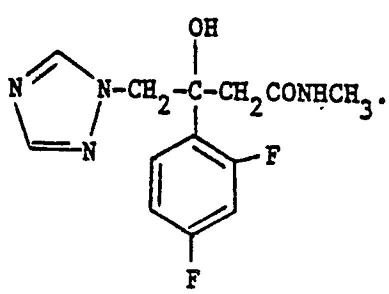

It had an m.p. of 129—131°.

*Analysis %:—*

| | | | |
|---|---|---|---|
| Found: | C, 52.8; | H, 4.9; | N, 19.3 |
| Calculated for $C_{13}H_{14}F_2N_4O_2$: | C, 52.7; | H, 4.8; | N, 18.9. |

Example 34

Preparation of 3-cyano-2-(2,4-dichlorophenyl)-1-(1H-1,2,4-triazol-1-yl)butan-2-ol (2 diastereoisomeric forms)

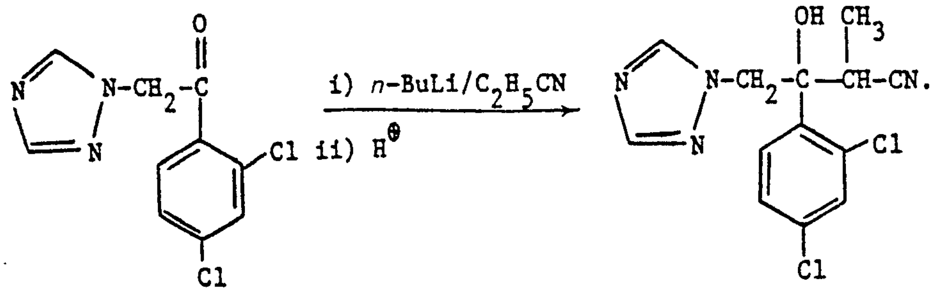


Propionitrile (1.21 g) in dry tetrahydrofuran (50 ml) was cooled to −72°. A solution of *n*-butyllithium as a solution in *n*-hexane (14.2 ml, 1.55 molar) was then slowly added whilst maintaining the temperature of the reaction mixture at −45° or below. After stirring for about 30 minutes, 2-(1H-1,2,4-triazol-1-yl)-2',4'-dichloroacetophenone (2.56 g) in dry tetrahydrofuran (THF) (50 ml) was added slowly with stirring over a 20 minute period, the temperature of the mixture being maintained at −70°. Stirring was continued at this temperature for 1 hour and then at −10° for ½ hour when glacial acetic acid (10 ml) in dry THF (15 ml) was added. The reaction mixture was then allowed to warm to room temperature (20°), was basified to pH 8 with solid sodium bicarbonate, and was extracted with ethyl acetate (3 × 75 ml). The combined organic extracts were washed three times with water, dried ($MgSO_4$), evaporated and ether (30 ml) was added to the residue, yielding a white crystalline solid and a yellow solution. The solid was filtered off, dissolved in a small volume of methylene chloride, and loaded onto an 18 g flash chromatography column of Merck's "Kieselgel 60" (Trade Mark) 230—400 mesh silica in ether (11 × 2 cm. diameter). Elution was carried out using 5% (by volume) acetone in ether at 1 p.s.i. "Diastereoisomer 1" of the title compound was eluted first, 0.79 g, m.p. 178—180°.

*Analysis %:—*

| | | | |
|---|---|---|---|
| Found: | C, 50.0; | H, 3.8; | N, 17.9 |
| Calculated for $C_{13}H_{12}Cl_2N_4O$: | C, 50.2; | H, 3.9; | N, 18.0. |

"Diastereoisomer 2" of the title compound was eluted next, 0.244 g, m.p. 202—205°.

*Analysis %:—*

| | | | |
|---|---|---|---|
| Found: | C, 50.4; | H, 3.9; | N, 17.6 |
| Calculated for $C_{13}H_{12}Cl_2N_4O$: | C, 50.2; | H, 3.9; | N, 18.0. |

Examples 35 and 36

The following compounds were prepared similarly to the previous Example, starting from the appropriate acetophenone, *n*-BuLi/$C_2H_5CN$ and glacial acetic acid:—

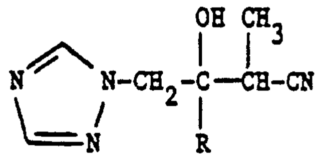

| Example No. | R | m.p. (°C) | Analysis % Theoretical in brackets C | H | N |
|---|---|---|---|---|---|
| 35 | 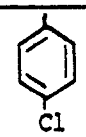 (mixture of diastereomers, not separated) | 159-162° | 56.4<br>(56.4 | 4.8<br>4.7 | 20.0<br>20.2) |
| 36 | 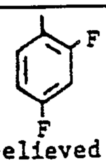 (believed to be a mixture of diastereomers, not separated) | 185-187° | 56.2<br>(56.1 | 4.3<br>4.3 | 20.0<br>20.1) |

Example 37

Preparation of 3-carbamoyl-2-(2,4-dichlorophenyl)-1-(1H-1,2,4-triazol-1-yl)butan-2-ol hemihydrate and 3-carboxy-2-(2,4-dichlorophenyl)-1-(1H-1,2,4-triazol-1-yl)butan-2-ol

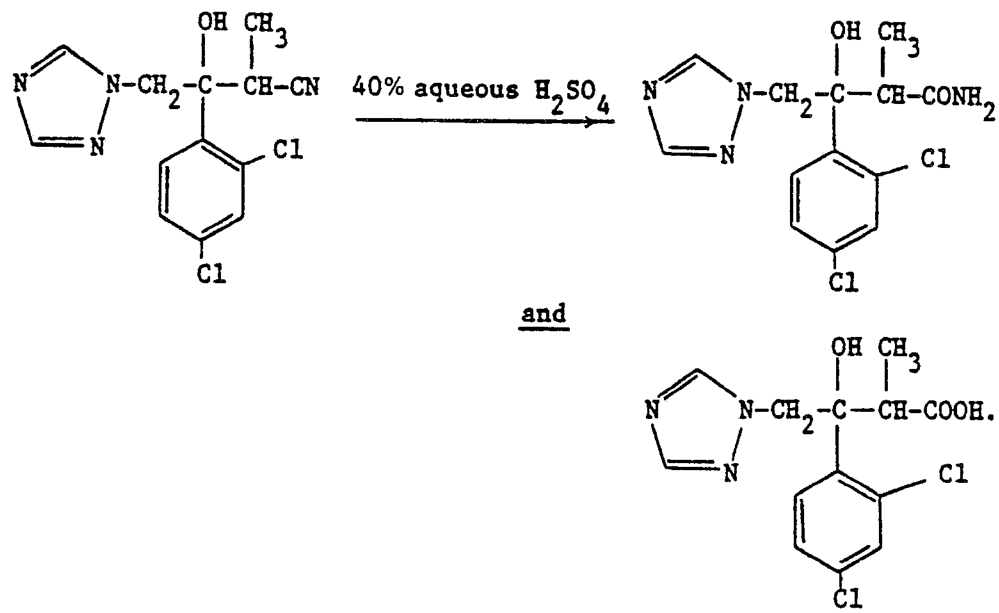


3-Cyano-2-(2,4-dichlorophenyl)-1-(1H-1,2,4-triazol-1-yl)butan-2-ol (700 mg, diastereoisomer 1 from the previous Example) was heated for 5½ hours at 90—95° in 40% (by volume) aqueous sulphuric acid. The solution was then stirred at room temperature (20°) for 19 hours, after which time saturated aqueous sodium bicarbonate solution was added to raise the pH to 8.0. The solution was then extracted with ethyl acetate (3 × 50 ml). The combined organic extracts were washed with water, dried ($MgSO_4$) and evaporated to yield the 3-carbamoyl title compound, 105 mg, m.p. 215—217° after trituration with ether.

*Analysis %:—* (3-carbamoyl compound)

Found: C, 46.8; H, 4.5; N, 15.5

Calculated for $C_{13}H_{14}Cl_2N_4O_2.\frac{1}{2}H_2O$: C, 46.2; H, 4.5; N, 15.6.

The aqueous phases resulting from the ethyl acetate extractions were combined, acidified to pH 2.0 with dilute hydrochloric acid, and extracted with ethyl acetate (3 × 50 ml). The combined organic extracts were washed with water, dried ($MgSO_4$) and evaporated to yield the title acid. After trituration with ether, the pure acid, 485 mg, m.p. 236—238°, was obtained.

*Analysis %:—*

Found: C, 47.0; H, 3.9; N, 12.4

Calculated for $C_{13}H_{13}Cl_2N_3O_3$: C, 47.3; H, 4.0; N, 12.7.

Example 38

3-Carbamoyl-2-(2,4-difluorophenyl)-1-(1H-1,2,4-triazol-1-yl)butan-2-ol $\frac{1}{4}$ hydrate, m.p. 170—172°, was prepared similarly to the previous Example by the hydrolysis of the corresponding nitrile prepared in Example 36 but using 80% aqueous sulphuric acid.

*Analysis %:—*

Found: C, 52.0; H, 4.8; N, 18.5

Calculated for $C_{13}H_{14}F_2N_4O_2.\frac{1}{4}H_2O$: C, 51.9; H, 4.8; N, 18.6.

Example 39

Preparation of 2-(4-chlorophenyl)-3-(carbamoyl)-1-(1H-1,2,4-triazol-1-yl)butan-2-ol

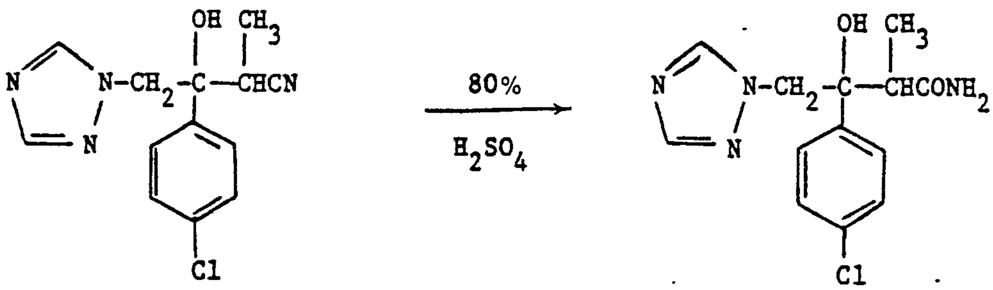


The mixture of diastereomeric nitriles from Example 35 (3.9 g) was heated in sulphuric acid (80%, 100 ml) for 4 hours at 60°. The reaction mixture was then cooled, diluted with water (200 ml), and calcium carbonate (50 g) was added in small portions with external cooling (ice bath). The mixture was then filtered, and the material which had been filtered off was washed well with water (200 ml) and methanol (200 ml). The washings were added to the filtrate, evaporated to dryness, and the residue extracted with ethyl acetate (3 × 100 ml). The extracts were combined, dried ($MgSO_4$), and evaporated to a white solid, 2.73 g. This material was absorbed onto silica ("Kiegelgel 60", 7 g) by dissolution in the minimum quantity of a chloroform:methanol mixture (5:1, v.v), addition of the silica, and evaporation of the solvents. This silica was added as a suspension in ether to a silica column ("Kieselgel 60", 25 g), and eluted with ether containing an increasing proportion of ethanol (2 → 10%). A proportion of the least polar amide diastereoisomer was eluted first in a pure state, and was recrystallised from ethyl acetate to give colourless crystals of one isomer of the title compound, m.p. 223—225°, 105 mg.

*Analysis %:—*

Found: C, 52.8; H, 5.3; N, 18.7

$C_{13}H_{15}ClN_4O_2$ requires: C, 53.0; H, 5.1; N, 19.0.

The remainder of the product was eluted as a mixture containing both the diastereoisomer characterised above and its more polar diastereomer (1:4 by n.m.r.). Recrystallisation from ethyl acetate gave colorless crystals, m.p. 186—189°, 404 mg.

*Analysis %:—*

Found: C, 53.0; H, 5.1; N, 19.4

$C_{13}H_{15}ClN_4O_2$ requires: C, 53.0; H, 5.1; N, 19.0.

Example 40

Preparation of 2-(2,4-dichlorophenyl)-3-(N-methylcarbamoyl)-1-(1H-1,2,4-triazol-1-yl)butan-2-ol

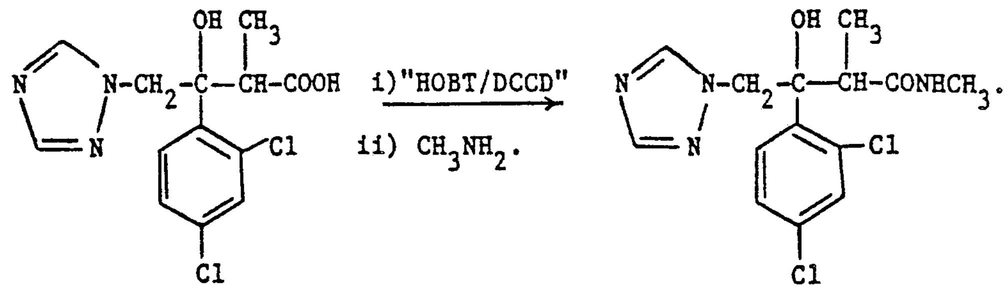


3-Carboxy-2-(2,4-dichlorophenyl)-1-(1H-1,2,4-triazol-1-yl)butan-2-ol (330 mg) was added to dry dioxan (10 ml) followed by 1-hydroxybenzotriazole hydrate ("HOBT") (203 mg) and dicyclohexylcarbodiimide ("DCCD") (618 mg). After stirring for 1 hour at room temperature (20°), methylamine (278 mg of 33% [by volume] solution in ethanol) was added and stirring was continued overnight (20 hours). After this time, the resulting precipitate of dicyclohexylurea was filtered off. The filtrate was added to water (50 ml) and solid sodium bicarbonate was added to pH 8.

The mixture was then extracted with ethyl acetate (3 × 50 ml) and the combined organic extracts were washed with water, dried ($MgSO_4$) and evaporated. The residue was dissolved in a small volume of methylene chloride and chromatographed on a Merck "Kieselgel 60" (Trade mark) silica flash column in ether. Elution with ether (100 ml) followed by 15% (by volume) ethanol in ether (300 ml) yielded, by collection of appropriate fractions, the title compound, 29 mg, m.p. 242—244°.

Since the filtered-off dicyclohexylurea contained a further quantity of the title compound, this was dissolved in a small amount of methanol and absorbed onto Merck's "Kieselgel 60" (Trade mark) silica (3 g), and the resulting slurry was then loaded onto a 10 g flash column of this material in ether. Elution with 10% (by volume) ethanol in ether, and collection of appropriate fractions followed by recrystallisation from isopropanol, yielded a further quantity of the title compound (81 mg).

*Analysis %:—*

| | | | |
|---|---|---|---|
| Found: | C, 48.9; | H, 4.8; | N, 16.2 |
| Calculated for $C_{14}H_{16}Cl_2N_4O_2$: | C, 49.0; | H, 4.7; | N, 16.3. |

Example 41

(A) Preparation of 2-(2,4-dichlorophenyl)-3-ethoxycarbonyl-3-methyl-1-(1H-1,2,4-triazol-1-yl)butan-2-ol and 4-(2,4-dichlorophenyl)-3,3-dimethyl-4-(1H-1,2,4-triazol-1-ylmethyl)-β-propiolactone

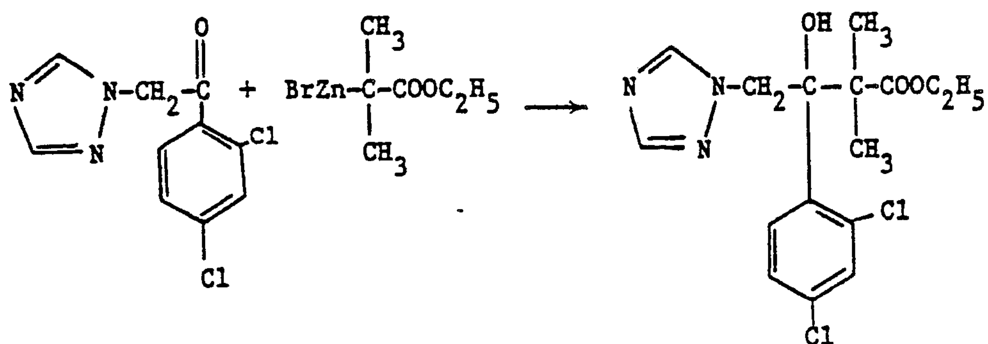

and

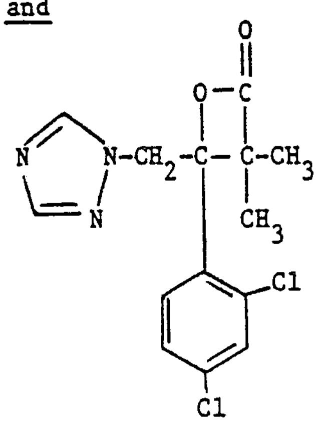

2-(1H-1,2,4-triazol-1-yl)-2',4'-dichloroacetophenone (2.56 g) in dry tetrahydrofuran (20 ml) and ethyl α-bromoisobutyrate (1.475 ml) in dry ether (10 ml) were added simultaneously to granulated zinc (1.5 g) in toluene (10 ml) over 20 minutes. The reaction mixture was then heated at 80° for 18 hours. The cooled reaction mixture was poured onto ice-cold sulphuric acid (0.2 N, 125 ml) and extracted with ether (200 ml). The ether extract was washed with brine, dried ($MgSO_4$), and concentrated *in vacuo*. The residue was flash chromatographed on silica (120 g) and eluted with 80% ethyl acetate/20% hexane. The initial fractions yielded the title ester, which was crystallised from ethyl acetate/hexane, yield of the pure product 61 mg, m.p. 95—96°.

*Analysis %:—*

| | | | |
|---|---|---|---|
| Found: | C, 51.7; | H, 5.2; | N, 11.1 |
| Calculated for $C_{16}H_{19}Cl_2N_3O_3$: | C, 51.6; | H, 5.1; | N, 11.3. |

The latter Fractions on evaporation gave the title β-lactone, which was recrystallised from ethyl acetate/hexane, yield of the pure product 240 mg, m.p. 177—178°.

*Analysis %:—*

| | | | |
|---|---|---|---|
| Found: | C, 51.8; | H, 3.9; | N, 12.8 |
| Calculated for $C_{14}H_{13}Cl_2N_3O_2$: | C, 51.5; | H, 4.0; | N, 12.9. |

(B) Preparation of 3-carbamoyl-2-(2,4-dichlorophenyl)-3-methyl-1-(1H-1,2,4-triazol-1-yl)butan-2-ol

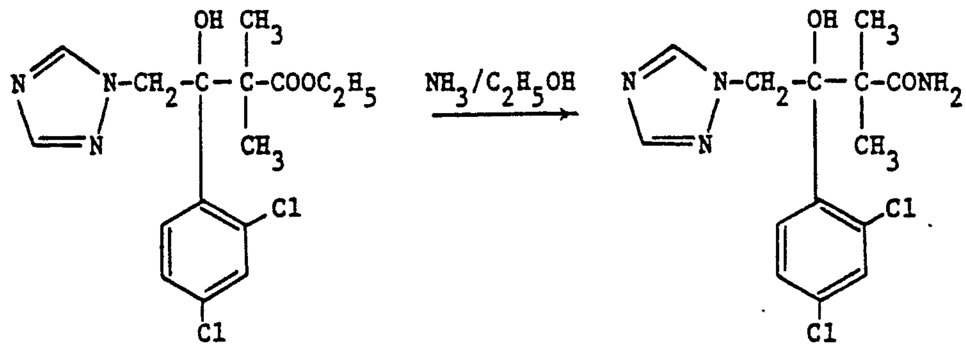


To a solution of 2-(2,4-dichlorophenyl)-3-ethoxycarbonyl-3-methyl-1-(1H-1,2,4-triazol-1-yl)butan-2-ol (75 mg) in ethanol (5 ml), aqueous ammonia aqueous ammonia (S.G. 0.88, 12 ml) was added and the solution was left at room temperature (20°) for 8 days. The solvent was then evaporated *in vacuo*, the residue was partitioned between methylene chloride and water, and the organic extracts were washed with brine and dried ($MgSO_4$). Removal of solvent followed by flash chromatography on silica (30 g) and elution with a mixture of methylene chloride/methanol/ammonia (93:7:1) gave the title compound, m.p. 162—3° (34.5 mg).

*Analysis %:—*

| | | | |
|---|---|---|---|
| Found: | C, 48.8; | H, 4.7; | N, 15.8 |
| Calculated for $C_{14}H_{16}Cl_2N_4O_2$: | C, 49.0; | H, 4.7; | N, 16.3. |

(C) 3-Carbamoyl-2-(2,4-dichlorophenyl)-1-(1H-1,2,4-triazol-1-yl)propan-2-ol was prepared similarly to parts (A) and (B) above from appropriate starting materials, and was confirmed spectroscopically to be identical to the product of Example 4.

(C) Preparation of 3-carbamoyl-2-(2,4-dichlorophenyl)-3-methyl-1-(1H-1,2,4-triazol-1-yl)butan-2-ol [alternative method to (B) above]

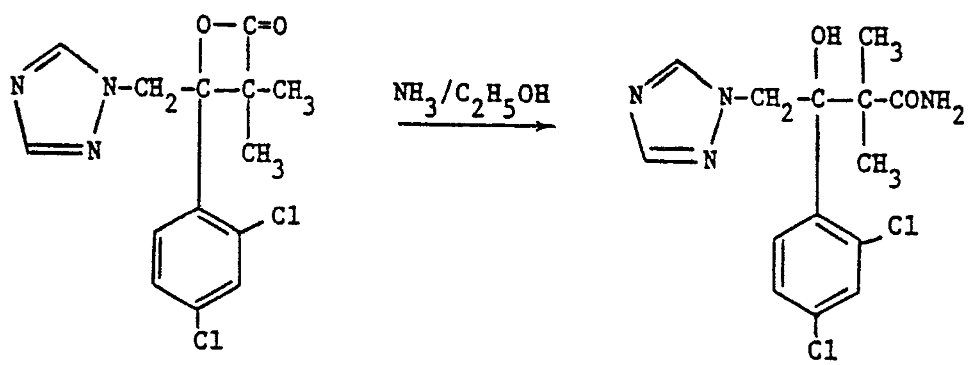


A solution of 4-(2,4-dichlorophenyl)-3,3-dimethyl-4-(1H-1,2,4-triazol-1-yl-methyl)-β-propiolactone (70

mg) in ethanol (4 ml) was treated with 0.88 ammonia (6 ml) and left to stand at room temperature for 5 days. The reaction mixture was then evaporated *in vacuo* and extracted and chromatographed by the method described in part (B) above to yield the title compound, m.p. 162—3°, (41 mg).

*Analysis %:—*

| | | | |
|---|---|---|---|
| Found: | C, 48.6; | H, 4.7; | N, 15.9 |
| Calculated for $C_{14}H_{16}Cl_2N_4O_2$: | C, 49.0; | H, 4.7; | N, 16.3. |

Example 42

Preparation of 2-(2,4-dichlorophenyl)-3-methyl-3-(N-methylcarbamoyl)-1-(1H-1,2,4-triazol-1-yl)butan-2-ol

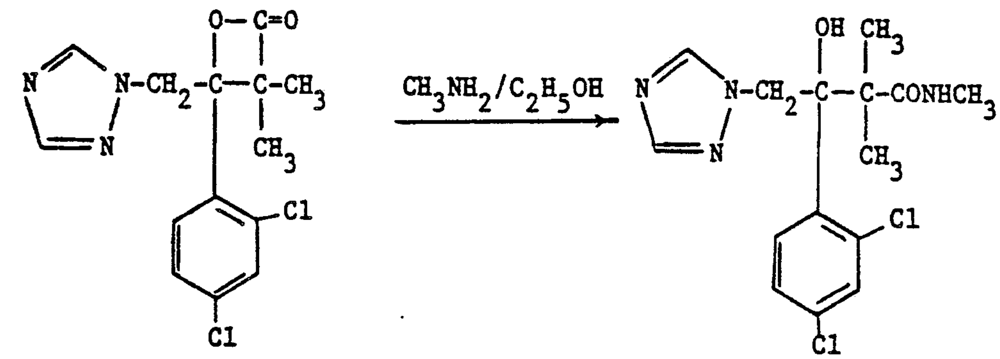


A solution of 4-(2,4-dichlorophenyl)-3,3-dimethyl-4-(1H-1,2,4-triazol-1-ylmethyl)-β-propiolactone (200 mg) in ethanol (5 ml) was treated with a solution of 35% (by volume) methylamine in ethanol (5 ml), and the resulting solution was left to stand overnight at room temperature (20°). After evaporating residual methylamine and ethanol, the residue was triturated with hexane and the resulting solid was crystallised from ethyl acetate/hexane to yield the title compound, m.p. 145—6°, (120 mg).

*Analysis %:—*

| | | | |
|---|---|---|---|
| Found: | C, 50.2 | H, 5.0; | N, 15.9 |
| Calculated for $C_{15}H_{18}Cl_2N_4O_2$: | C, 50.4; | H, 5.0; | N, 15.7. |

Example 43

(A.) Preparation of 4-(2,4-dichlorophenyl)-4-(1H-1,2,4-triazol-1-ylmethyl)-β-propiolactone

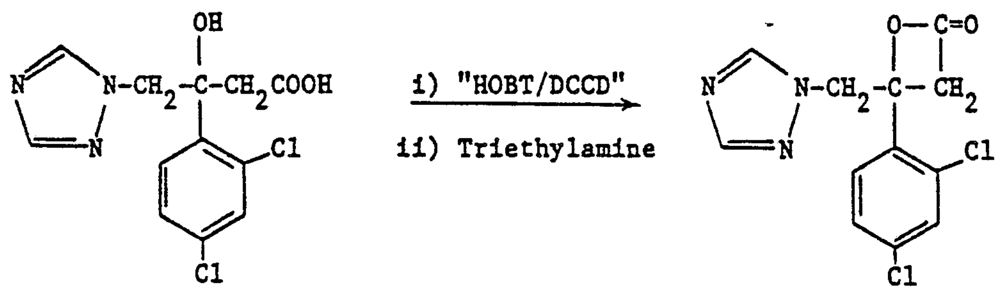


3-Carboxy-2-(2,4-dichlorophenyl)-1-(1H-1,2,4-triazol-1-yl)butan-2-ol (948 mg) was dissolved in dry dioxan (20 ml) and 1-hydroxybenzotriazole hydrate (0.61 g) followed by dicyclohexylcarbodiimide (1.85 g), were then added. The resulting mixture was stirred at room temperature (20°) for 2 hours when triethylamine (455 mg) was added and stirring was continued overnight (19 hours). The mixture was then added to water (100 ml) and extracted with ethyl acetate (3 × 50 ml). The precipitate of dicyclohexylurea was filtered off after the first extraction. The combined organic extracts were washed with water, dried ($MgSO_4$) and evaporated. The resulting residue was dissolved in a little methylene chloride and loaded onto a flash column of Merck's "Kieselgel 60" (Trade mark) silica (12 g, 230—400 mesh) in ether. Elution with ether (100 ml) and then with 5% (by volume) acetone in ether (300 ml) gave, after collection of appropriate fractions, the title compound, 600 mg, m.p. 178—180°.

*Analysis %:—*

| | | | |
|---|---|---|---|
| Found: | C, 48.1; | H, 3.0; | N, 14.0 |
| Calculated for $C_{12}H_9Cl_2N_3O_2$: | C, 48.4; | H, 3.0; | N, 14.1. |

(B.) Preparation of 3-(N-methylcarbamoyl)-2-(2,4-dichlorophenyl)-1H-(1,2,4-triazol-1-yl)propan-2-ol

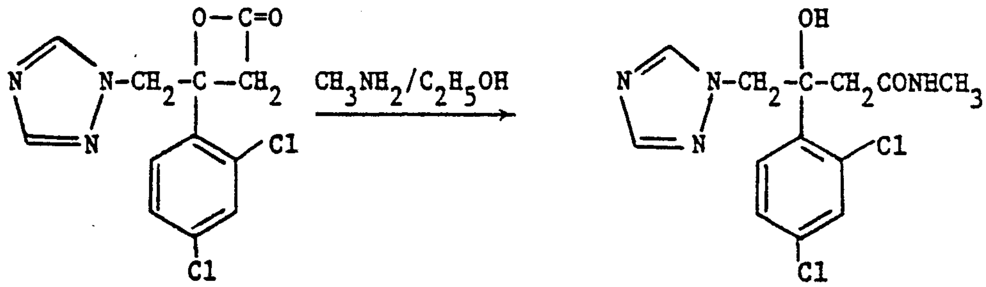


This reaction was carried out similarly to Example 42 using the starting materials specified in the reaction scheme to give the title compound, confirmed spectroscopically to be the desired product and to be identical to the product of Example 5.

Example 44

Preparation of 3-carbamoyl-2-(2,4-dichlorophenyl)-1H-(1,2,4-triazol-1-yl)propan-1-ol

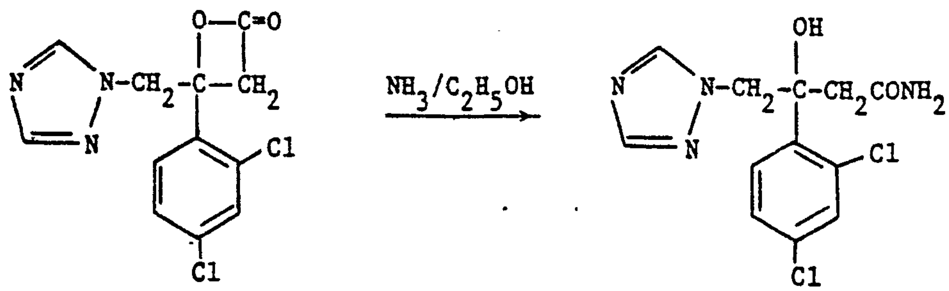


This reaction was carried out similarly to Example 41(C) using the starting materials specified in the reaction sequence to give the title compound, confirmed spectroscopically to be the desired product and to be identical to the product of Example 4.

Example 45

Preparation of 3-(N-acetylcarbamoyl)-2-(2,4-dichlorophenyl)-1-(1H-1,2,4-triazol-1-yl)propan-2-ol hemihydrate

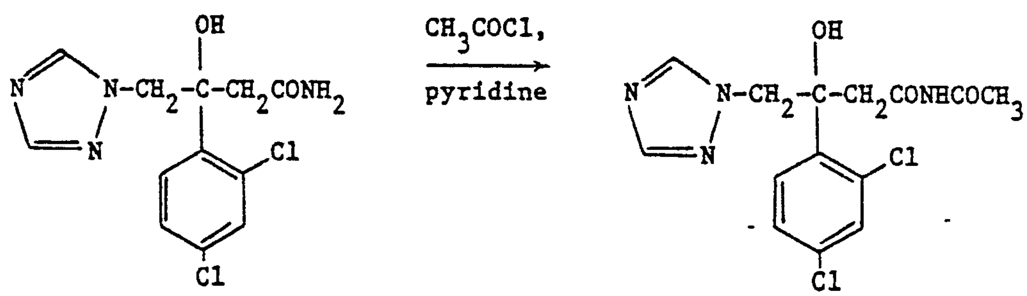


A solution of acetyl chloride (0.12 g, 1.5 mMole) in dry acetonitrile (2 ml) was added dropwise to a stirred solution of 3-carbamoyl-2-(2,4-dichlorophenyl)-1-(1H-1,2,4-triazol-1-yl)propan-2-ol (0.314 g, 1 mMole) and pyridine (0.12 g, 1.5 mMole) in dry acetonitrile (2 ml) at −20°. The solution was allowed to warm to room temperature and was stirred at room temperature for a further 18 hours. The solvent was then removed under reduced pressure and the residue was partitioned between water (10 ml) and chloroform (40 ml). The organic phase was separated, dried over $MgSO_4$ and evaporated. The residue was chromatographed on silica eluting with 10:1 (v:v) chloroform/methanol and the fractions containing the product were combined, evaporated, and recrystallised from diisopropyl ether to furnish the title compound, (67 mg, 19%), m.p. 148—50°.

*Analysis %:—*

| | | | |
|---|---|---|---|
| Found: | C, 45.8; | H, 3.9; | N, 15.0 |
| Calculated for $C_{14}H_{14}N_4O_3Cl_2.\frac{1}{2}H_2O$: | C, 45.9; | H, 4.1; | N, 15.3. |

Examples 46 and 47

The following compounds were prepared similarly to Example 45, starting from the same amide, pyridine, and the appropriate acid chloride of the formula $R^3$.Cl:—

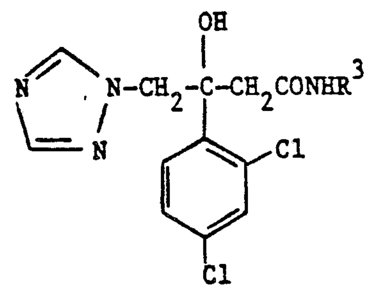

| Example No. | $R^3$ | m.p. (°C) | Analysis % (Theoretical in brackets) C | H | N |
|---|---|---|---|---|---|
| 46 | 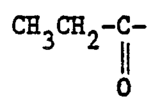 | 101-3° | 48.2<br>(48.5 | 4.3<br>4.3 | 14.7<br>15.1) |
| 47 | 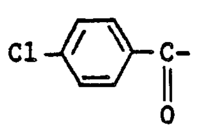 | 183-4° | 50.3<br>(50.3 | 3.4<br>3.3 | 12.1<br>12.3) |

Example 48

Preparation of 2-(2,4-dichlorophenyl)-3-(N,N-dimethoxycarbonylcarbamoyl)-1-(1H-1,2,4-triazol-1-yl)-propan-2-ol

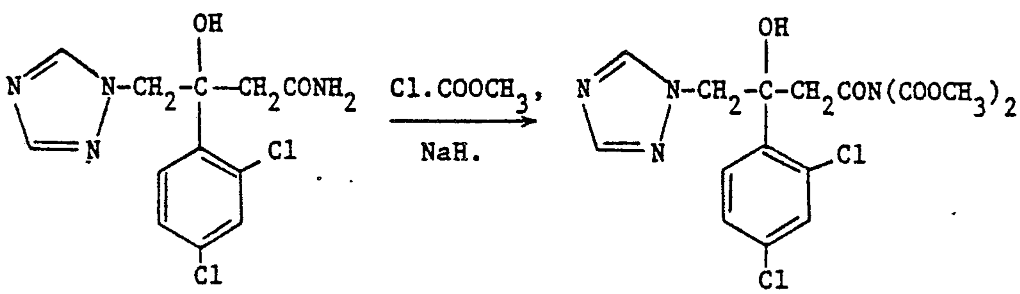


3-Carbamoyl-2-(2,4-Dichlorophenyl)-1-(1H-1,2,4-triazol-1-yl)propan-2-ol (0.94 g, 3 mMole) was added to a suspension of oil-free sodium hydride (0.14 g, 5.8 mMole) in dry tetrahydrofuran (10 ml). The suspension was stirred for 1 hour and then a solution of methyl chloroformate (0.6 g, 6.3 mMole) in dry tetrahydrofuran (10 ml) was added dropwise over 15 minutes. The mixture was stirred at room temperature for 3 hours and then the solvent was evaporated *in vacuo*. The residue was partitioned between saturated aqueous sodium bicarbonate solution (10 ml) and ethyl acetate (40 ml), and the organic phase was separated, washed with brine (10 ml), dried ($MgSO_4$) and evaporated. The residue was chromatographed on silica eluting with ethyl acetate/methanol 95:5 (v;v), and fractions containing the product were combined and evaporated and recrystallized from ethyl acetate-hexane to give the title compound, (62 mg, 5%), m.p. 155—6°.

*Analysis %:—*

| | | | |
|---|---|---|---|
| Found: | C, 44.5; | H, 3.7; | N, 13.2 |
| Calculated for $C_{16}H_{16}N_4O_6Cl_2$: | C, 44.5; | H, 3.7; | N, 13.0. |

Example 49

Preparation of 1-carbamoyl-2-(2,4-dichlorophenyl)-3-(1H-1,2,4-triazol-1-yl)propan-2-ol

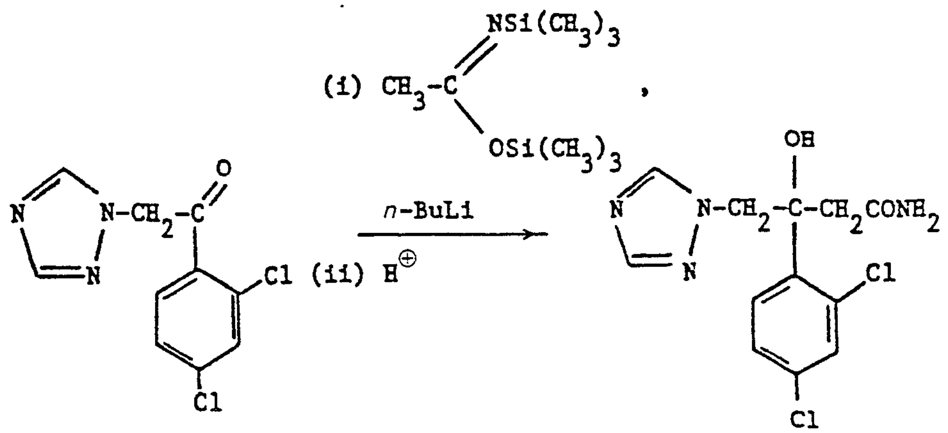


Bis(trimethylsilyl)acetamide (1.99 g) was stirred at −70° in dry tetrahydrofuran (15 ml) whilst n-butyllithium in hexane (6.3 ml, 1.55 M) was added dropwise over ten minutes. The resulting solution was stirred at −70° for 30 minutes, then a solution of 2-(1H-1,2,4-triazol-1-yl)-2′,4′-dichloroacetophenone (1.0 g) in dry tetrahydrofuran (10 ml) was added dropwise over 10 minutes, and the mixture was stirred for 1½ hours at −70°. The reaction mixture was then allowed to warm to room temperature, and water (5 ml) and hydrochloric acid (7 ml, 2N) were added.

The mixture was then adjusted to pH 8 by the addition of solid sodium bicarbonate, and then extracted with ethyl acetate (3 × 10 ml). The combined extracts were washed with saturated sodium chloride solution (3 × 10 ml), dried ($MgSO_4$), and evaporated to a gum, 1.1 g.

This gum was chromatographed on silica ("Kieselgel 60", Merck), eluting with ether containing 5% by volume ethanol. After the elution of unreacted ketone, the product eluted, and the fractions containing this material were combined and evaporated to give the pure title compound, (0.21 g), confirmed spectroscopically to be identical to the product of Example 4.

Example 50

Preparation of 1-carbamoyl-2-(2,4-dichlorophenyl)-3-(1H-1,2,4-triazol-1-yl)propan-2-ol

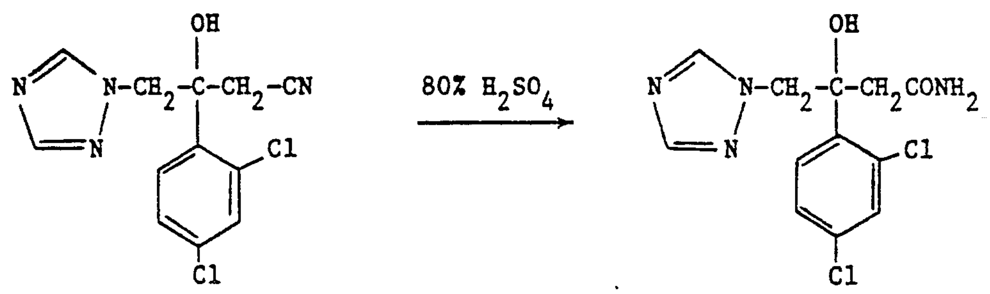


1-Cyano-2-(2,4-dichlorophenyl)-3-(1H-1,2,4-triazol-1-yl)propan-2-ol (1.0 g) was heated at 60° for 2½ hours in sulphuric acid (10 ml, 80%). The reaction mixture was then cooled to room temperature, carefully treated with water (100 ml), and adjusted to pH 9 with solid sodium hydroxide. The resulting solution was extracted with methylene chloride (3 × 50 ml), and the combined extracts were evaporated to a gum, which was chromatographed on silica ("Kieselgel 60", Merck), eluting with methylene chloride containing 3% by volume methanol, increasing to 6% methanol. The fractions which contained the product as judged by t.l.c. were combined and evaporated to a white solid, 0.91 g. This was dissolved in a mixture of acetone and methylene chloride at reflux and the product was precipitated by the addition of hexane to give fine crystals, m.p. 144—145.5, 0.61 g, confirmed spectroscopically to be identical with the product of Example 4 after drying under vacuum for 7 hours at 80°.

Example 51

Preparation of 2-(2,4-dichlorophenyl)-1-{N-(2-[methylsulphinyl]ethyl)carbamoyl}-3-(1H-1,2,4-triazol-1-yl)-propan-2-ol

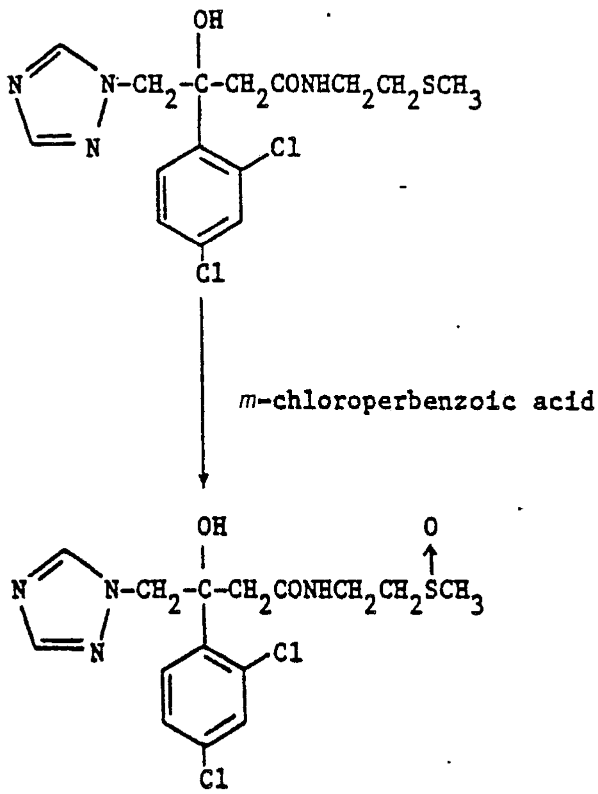


2-(2,4-Dichlorophenyl)-1-{N-(2-[methylthio]ethyl)carbamoyl}-3-(1H-1,2,4-triazol-1-yl)propan-2-ol (0.8 g) and *m*-chloroperbenzoic acid (85%, 0.35 g, 1 equiv.) were stirred at room temperature in a mixture of isopropanol and methylene chloride (1:1, v/v, 40 ml) for two days. The solvents were then removed under reduced pressure, and the residue was chromatographed on silica (Merck, "Kieselgel 60", 25 g), eluting with a mixture of chloroform, methanol and ammonia (S.G. 0.880) (160:20:5, v/v). A portion of the isomer which was eluted first was obtained pure, 116 mg, m.p. 168—170°.

*Analysis %:—*

| | | | |
|---|---|---|---|
| Found: | C, 44.1; | H, 4.4; | N, 13.5 |
| $C_{15}H_{18}Cl_2N_4O_3S$ requires: | C, 44.4; | H, 4.5; | N, 13.8. |

The bulk of the material eluted as a mixture containing both diastereoisomers (330 mg). This material was used in the preparation of the sulphone which follows.

Example 52

Preparation of 2-(2,4-dichlorophenyl)-1-{N-(2-[methylsulphonyl]ethyl)carbamoyl}-3-(1H-1,2,4-triazol-1-yl)-propan-2-ol

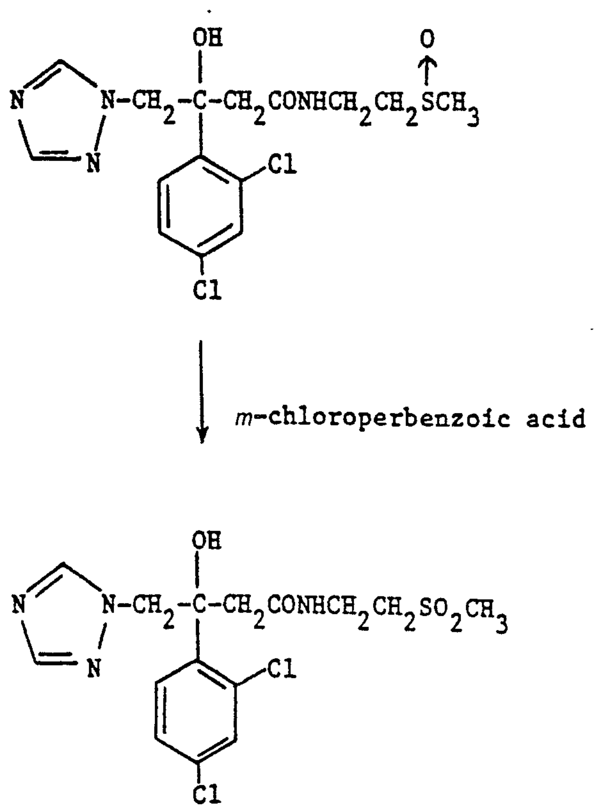


The unseparated mixture of diastereoisomers from the previous Example (330 mg) and *m*-chloroperbenzoic acid (140 mg) were stirred together in a mixture of isopropanol and methylene chloride (1:1, v/v, 20 ml) at 0°. After one hour at 0°, the reaction mixture was allowed to reach room temperature and was stirred overnight. The solvents were then removed under reduced pressure, and the residue was dissolved in ethyl acetate (50 ml). The resulting solution was washed with saturated sodium bicarbonate solution (2 × 20 ml), then with saturated sodium chloride solution (2 × 20 ml), dried ($MgSO_4$), and evaporated to a gum which was triturated with diisopropyl ether to give a white solid, 209 mg, m.p. 123—124°.

*Analysis %:—*

| | | | |
|---|---|---|---|
| Found: | C, 42.6; | H, 4.3; | N, 13.2 |
| $C_{15}H_{18}Cl_2N_4O_4S$ requires: | C, 42.8; | H, 4.3; | N, 13.3. |

Example 53

Preparation of 1-aziridinylcarbonyl-2-(2,4-dichlorophenyl)-3-(1H-1,2,4-triazol-1-yl)propan-2-ol

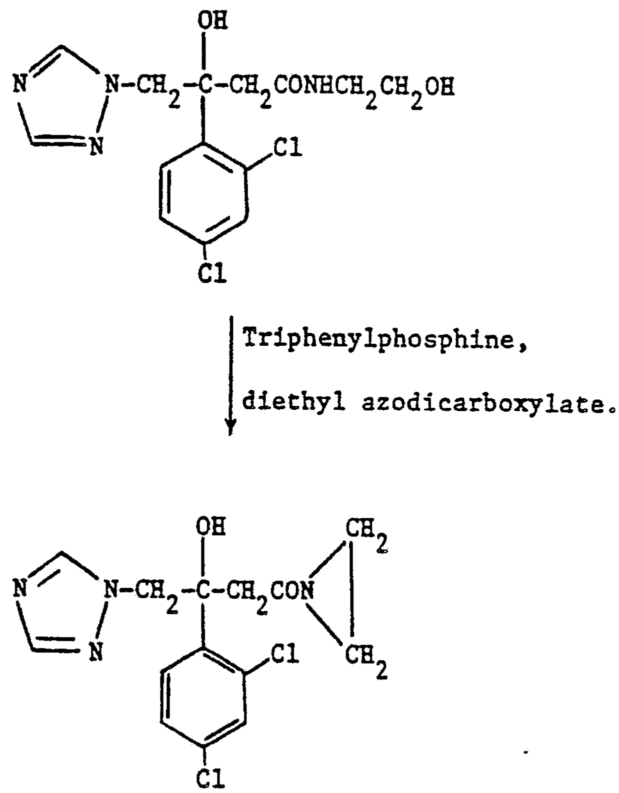


1-(N-[2-Hydroxyethyl]carbamoyl)-2-(2,4-dichlorophenyl)-3-(1H-1,2,4-triazol-1-yl)propan-2-ol (1.0 g), triphenylphosphine (1.09 g) and diethyl azodicarboxylate (0.72 g) were stirred at room temperature for 20 hours in dry tetrahydrofuran (20 ml). The reaction mixture was then diluted with water (70 ml) and extracted with ethyl acetate (3 × 50 ml). The combined organic extracts were washed with saturated sodium chloride solution (2 × 20 ml), dried ($MgSO_4$), and evaporated to a brown gum. This material was chromatographed on Merck "Kieselgel 60" silica, eluting with 5% by volume ethanol in ether increasing to 10% ethanol in ether. The eluted material, which was one compound as judged by t.l.c., was crystallised from ethyl acetate/*n*-pentane to give colourless crystals of the title compound, 441 mg, m.p. 151—153°.

*Analysis %:—*

| | | | |
|---|---|---|---|
| Found: | C, 49.2; | H, 4.0; | N, 16.3 |
| $C_{14}H_{14}Cl_2N_4O_2$ requires: | C, 49.3; | H, 4.1; | N, 16.4. |

Example 54

Preparation of 2-(2,4-dichlorophenyl)-1-thiocarbamoyl-3-(1H-1,2,4-triazol-1-yl)propan-2-ol

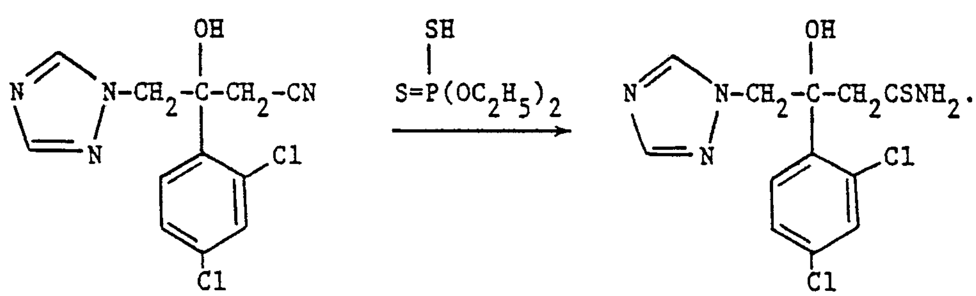


A mixture of 3-cyano-2-(2,4-dichlorophenyl)-1-(1H-1,2,4-triazol-1-yl)propan-2-ol (250 mg), O,O-diethyl dithiophosphoric acid (0.5 ml) and water (0.1 ml) was heated on steam bath for 3 hours, evaporated under reduced pressure to an oil, and chromatographed on "Merck 60H" (Trademark) silica (10 g) eluting with ethyl acetate. The eluted product, after evaporation, was triturated under petroleum ether (b.p. 40—60°) to give the title compound as a yellow solid (143 mg), m.p. 158°—159°.

*Analysis %:—*
Found: C, 43.8; H, 3.6; N, 16.9
Calculated for $C_{12}H_{12}Cl_2N_4OS$: C, 43.6; H, 3.6; N, 16.9.

Example 55

The following illustrate pharmaceutical compositions for the treatment of fungal infections:—

(a) *Capsule:* 71 parts by weight of the compound of Example 1 or 2 are granulated with 3 parts maize starch and 22 parts lactose and then a further 3 parts maize starch and 1 part magnesium stearate are added. The mixture is regranulated and filled into hard gelatin capsules.

(b) *Cream:* 2 parts by weight of the compound of Example 3 are dissolved in 10 parts of propylene glycol and mixed into 88 parts of a vanishing cream base.

(c) *Pessary:* 2 parts by weight of the compound of Example 5 are suspended in 98 parts of a warm liquified suppository base which is poured into moulds and allowed to solidify.

The following Preparations, in which all temperatures are in °C, illustrate the preparation of certain starting materials:—

Preparation 1

Preparation of 1-carboxy-2-(2,4-dichlorophenyl)-3-(1H-1,2,4-triazol-1-yl)propan-2-ol

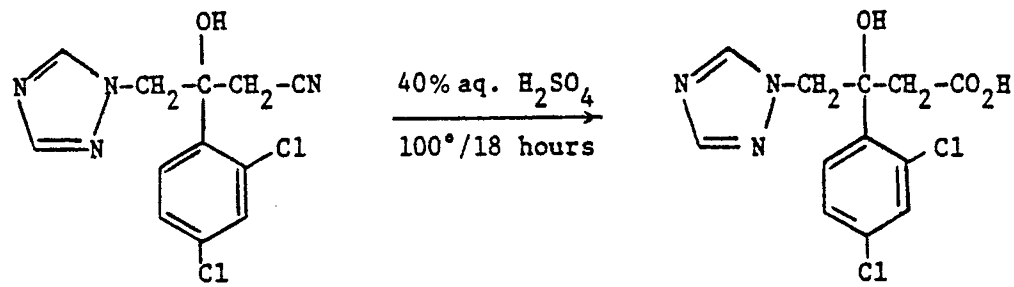


1-Cyano-2-(2,4-Dichlorophenyl)-3-(1H-1,2,4-triazol-1-yl)-2-propanol (4 g, 13.9 mmole) was dissolved in 40% aqueous sulphuric acid (100 ml) and heated in an oil bath at 100—110° for 18 hours. The solution was then cooled, diluted with water (200 ml), and rendered alkaline by the slow addition of solid sodium bicarbonate. The mixture was then extracted several times with ethyl acetate (3 × 100 ml) and the aqueous phase was rendered acidic (pH 3) by the addition of dilute orthophosphoric acid. The aqueous phase was then extracted with ether (3 × 150 ml), the combined ether extracts were washed once with water, and then dried over magnesium sulphate. Evaporation of the ether gave the title compound as a pale yellow solid, 2.7 g, (62%), m.p. 158—9°.

*Analysis %:—*
Found: C, 46.35 H, 3.5; N, 13.6
Required for $C_{12}H_{11}Cl_2N_3O_3$: C, 45.6; H, 3.5; N, 13.3.

Also prepared similarly was 1-carboxy-2-(2,4-difluorophenyl)-3-(1H-1,2,4-triazol-1-yl)propan-2-ol, m.p. 185—7°.

*Analysis %:—*
Found: C, 50.8; H, 3.9; N, 14.8
Calculated for $C_{12}H_{11}F_2N_3O_3$: C, 51.0; H, 3.9; N, 14.8.

Preparation 2

Preparation of 3-(2,4-dichlorophenyl)-3-hydroxy-4-(1H-1,2,4-triazol-1-yl)butyrimidic acid, ethyl ester dihydrochloride

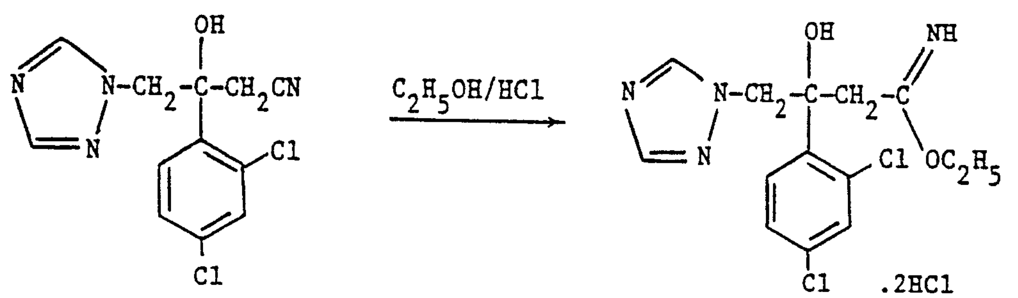


1-Cyano-2-(2,4-dichlorophenyl)-3-(1H-1,2,4-triazol-1-yl)propan-2-ol (1 g) was dissolved in dry ethyl alcohol (100 ml) and dry hydrogen chloride gas was bubbled in, at 0°, for 10 minutes. The reaction mixture

was then stirred at room temperature overnight, and then the solvent was decanted from the solid. The solid was then washed with dry ether and dried to yield the title compound (1.15 g), m.p. 154—156°. The product was used in Example 4.

*Analysis %:—*

| | | | |
|---|---|---|---|
| Found: | C, 40.6; | H, 4.4; | N, 13.6 |
| Calculated for $C_{14}H_{16}Cl_2N_4O_2.2HCl$: | C, 40.4; | H, 4.4; | N, 13.5. |

Preparation 3

(i) Preparation of 2-(1H-1,2,4-Triazol-1-yl)-2',4'-dichloroacetophenone (Y)

This compound was prepared similarly to the method described in British Patent Specification No. 1512918:—

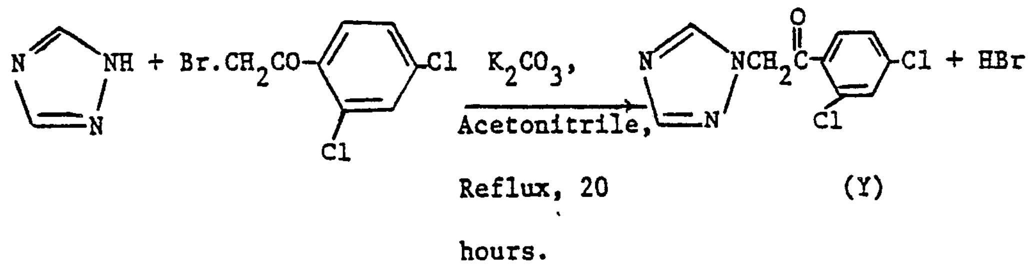


(Y)

(ii) Preparation of 2-(2,4-Dichlorophenyl)-2-(1H-1,2,4-triazol-1-ylmethyl)-oxirane (Z)

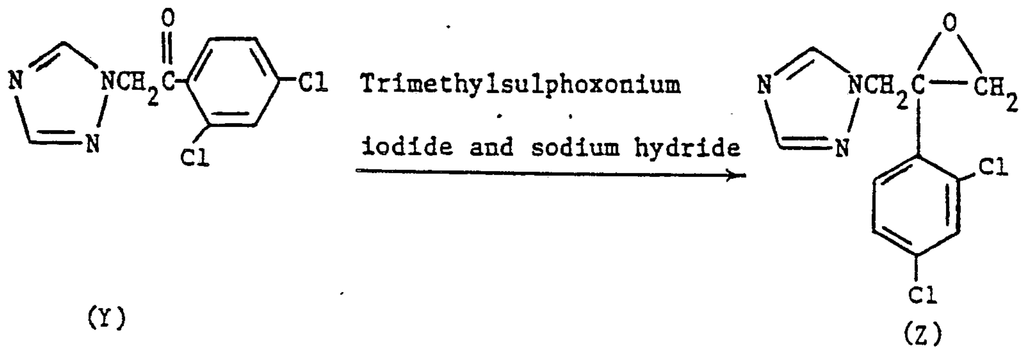


(Y) (Z)

3.78 g (0.079 Mole) of sodium hydride (50% dispersion in oil) was suspended, with stirring, in 20 ml of dry ether. The ether was then removed by decantation, and the sodium hydride was dried in a stream of dry nitrogen. 100 Ml of dry dimethyl sulphoxide was added followed by 17.34 g (0.079 mole) of dry powdered trimethylsulphoxonium iodide, in portions, over 15 minutes. The resulting mixture was stirred for 30 minutes at room temperature (20°C). 18.33 g (0.072 Mole) of compound (Y) as a solution in 50 ml of dry dimethyl sulphoxide was then added. The mixture was heated at 60°C for 3 hours and then stood at room temperature overnight. The reaction mixture was cooled and quenched in ice. The product was then extracted into ethyl acetate (600 ml). The ethyl acetate layer was separated, dried over magnesium sulphate, and concentrated to give a red gum. Column chromatography of the gum on silica, eluting with ether, gave the product (Z). On evaporation, 6.62 g (34.4%) of the title compound (Z) was obtained as a gum which solidified on trituration. The pure product melted at 57—59°.

*Analysis %:—*

| | | | |
|---|---|---|---|
| Found: | C, 48.6; | H, 3.3; | N, 15.3 |
| Calculated for $C_{11}H_9Cl_2N_3O$: | C, 49.0; | H, 3.4; | N, 15.5. |

Preparation 4

(A) Preparation of 2-chloro-2',4'-difluoroacetophenone

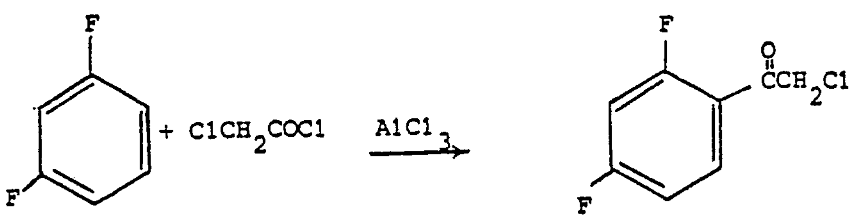


Chloroacetyl chloride (113 g, 1.0 M) was added dropwise to a stirred mixture of 1,3-difluorobenzene

(114 g, 1.0 M) and anhydrous aluminium chloride (146.6 g, 1.1 M) at room temperature (20°). The mixture was stirred for a further five hours at 50°—55°C. Methylene chloride (48.5 ml) was added slowly as the mixture was allowed to cool to room temperature. The methylene chloride layer was separated, washed with water (2 × 320 ml) and the solvent removed by distillation at reduced pressure leaving a pale yellow solid (180 g).

A portion of the crude product (145 g) was crystallised from n-hexane (435 ml) giving the title compound (113 g, 73%) m.pt. 47°—49°C (literature* 46.5°C). IR (KBr) and nmr ($CDCl_3$) were consistent with the desired structure.

(B) Preparation of 2',4'-difluoro-2-(1H-1,2,4-triazol-1-yl)acetophenone hydrochloride

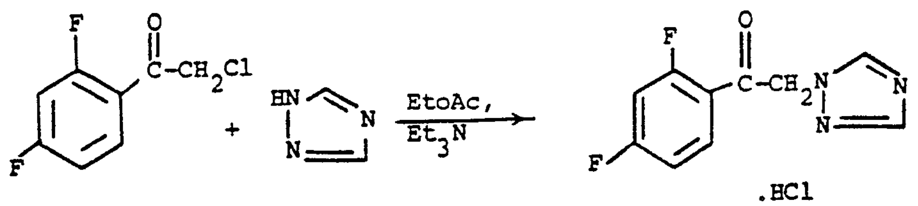


To a mixture of 1,2,4-triazole (30.4 g, 0.44 M) and triethylamine (15.1 g, 0.15 M) in refluxing ethyl acetate (186 ml) was added a solution of 2-chloro-2',4'-difluoroacetophenone (38.1 g, 0.2 M) in ethyl acetate (80 ml). The mixture was refluxed for six hours then cooled to room temperature and the insolubles were removed by filtration. The filtrate was washed with water (2 × 200 ml) and then the solvent was removed by distillation at reduced pressure. The crude product was dissolved in ethyl acetate (150 ml) then 25% w/v HCl gas in isopropanol was added. The mixture was granulated at 0°C for one hour and then the solid was collected by filtration and dried to give the title compound (21.6 g, 40%), melting point 167°—170°C. IR (KBr) and nmr (DMSO) were consistent with the desired structure.

This intermediate was characterised as the free base, which was prepared by the following technique:—

To stirred slurry of sodium bicarbonate (16.8 g, 0.2 M) and 1,2,4-triazole (27.6 g, 0.4 M) in refluxing toluene (180 ml) was added a solution of 2-chloro-2',4'-difluoroacetophenone (38.1 g, 0.2 M) in toluene (45 ml). The mixture was stirred at reflux for three hours and the water formed during the reaction was removed using a Dean and Stark trap. The reaction mixture was cooled to room temperature and then water (180 ml) was added. The toluene layer was separated and the solvent removed by distillation at reduced pressure. The resulting pale brown solid was crystallised from 1:1 ethyl acetate: *n*-hexane (70 ml) giving the title compound (3.9 g), melting point 103°—105°C. The IR (KBr) and nmr ($CDCl_3$) were consistent with the desired structure.

*Analysis %:—*

| | | | |
|---|---|---|---|
| Found: | C, 53.6; | H, 3.15; | N, 18.7 |
| Calculated for $C_{10}H_7F_2N_3O$: | C, 53.8; | H, 3.2; | N, 18.8. |

For 4'-chloro-2-(1H-1,2,4-triazol-1-yl) acetophenone see DT—OS 2431407.

Activity Data

In mice, $PD_{50}$ (oral) values in mg/kg:—

| Product of Example No. | $PD_{50}$ (mg/kg) |
|---|---|
| 1 | 1.3 |
| 2 | 1.3 |
| 3 | ~40 |
| 4 | 0.2 |
| 5 | 0.2 |
| 6 | ~20 |
| 7 | 0.4 |

* Von D. Ehlers, H. Bercher and A. Grisk, *J. Prakt, Chem., 315,* 1169 (1973).

| Product of Example No. | $PD_{50}$ (mg/kg) |
|---|---|
| 8 | 0.1 |
| 9 | 0.4 |
| 10 | 0.1 |
| 11 | ~30 |
| 12 | 3.1 |
| 13 | ~40 |
| 14 | 0.4 |
| 15 | 0.2 |
| 16 | ~20 |
| 17 | 1.5 |
| 18 | 0.4 |
| 19 | 2.2 |
| 20 | 0.1 |
| 21 | 0.6 |
| 22 | 0.2 |
| 23 | 0.2 |
| 24 | 0.4 |
| 25 | 3.5 |
| 26 | 2.2 |
| 27 | 4.2 |
| 28 | 4.2 |
| 29 | 4.2 |
| 30 | 0.1 |
| 31 | 3.1 |
| 32 | 0.2 |
| 33 | 0.3 |
| 34 | 0.1 |
| 35 | 2.0 |
| 36 | 10 |
| 37 | 0.4 |
| 38 | 0.4 |
| 39 | 0.5 |

| Product of Example No. | $PD_{50}$ (mg/kg) |
|---|---|
| 40 | 0.2 |
| 41 | 3.1 |
| 42 | 0.6 |
| 43(B) | 0.2 |
| 44 | 0.2 |
| 45 | 0.1 |
| 46 | 0.1 |
| 47 | 0.1 |
| 48 | ~0.3 |
| 49 | 0.2 |
| 50 | 0.2 |
| 51 (isomer melting at 168—170°) | 0.2 |
| 52 | 0.1 |
| 53 | 4.2 |
| 54 | 0.5 |

**Claims for the Contracting States: BE CH DE FR GB IT LI LU NL SE**

1. Compounds of the formula:—

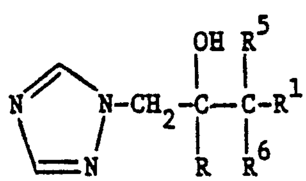

— (I)

where

R is phenyl optionally substituted by 1 to 3 substituents each independently selected from F, Cl, Br, I, $CF_3$, $C_1$—$C_4$ alkyl and $C_1$—$C_4$ alkoxy, or R is a 5-chloropyrid-2-yl group;

$R^1$ is —CN, —$CSNH_2$, or —$CONR^2R^3$ where either

(a) $R^2$ is H or $C_1$—$C_6$ alkyl, and $R^3$ is H, $C_1$—$C_6$ alkyl, benzyl, phenethyl, phenyl, —$CH_2CF_3$, adamantyl, pyridylmethyl, $C_3$—$C_7$ cycloalkyl, carbamoylmethyl, ($C_2$—$C_4$ alkenyl)methyl, 2-hydroxyethyl, 2-(dimethylamino)ethyl, 2-(methylthio)ethyl, 2-(methylsulphinyl)ethyl, 2-(methylsulphonyl)ethyl or 2-phenoxyethyl; said benzyl, phenethyl, phenyl and phenoxy groups being optionally ring-substituted by 1 or 2 substituents each independently selected from $C_1$—$C_4$ alkyl, $C_1$—$C_4$ alkoxy, F, Cl, Br, I and $CF_3$,

(b) $R^2$ and $R^3$ taken together with the nitrogen atom to which they are attached represent a group of the formula:—

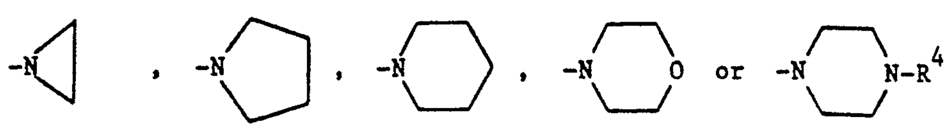

where

$R^4$ is H, $C_1$—$C_4$ alkyl, $C_2$—$C_4$ alkanoyl or ($C_1$—$C_4$ alkoxy)carbonyl;

(c) $R^2$ is H or $C_1$—$C_4$ alkyl and $R^3$ is $C_2$—$C_4$ alkanoyl or benzoyl, said benzoyl group being optionally substituted by 1 or 2 substituents each independently selected from F, Cl, Br, I, $CF_3$, $C_1$—$C_4$ alkyl and $C_1$—$C_4$ alkoxy); or

(d) $R^2$ and $R^3$ are both ($C_1$—$C_4$ alkoxy)carbonyl; $R^5$ is H or $CH_3$; and $R^6$ is H or $CH_3$; and their pharmaceutically acceptable salts.

2. A compound as claimed in claim 1 wherein R is phenyl substituted by 1 to 3 substituents each independently selected from F, Cl, Br, I and $CF_3$.

3. A compound as claimed in claim 2, wherein R is 4-fluorophenyl, 4-chlorophenyl, 4-bromophenyl, 4-iodophenyl, 4-trifluoromethylphenyl, 2-chlorophenyl, 2,4-dichlorophenyl, 2,4-difluorophenyl, 2,5-difluorophenyl, 2-fluoro-4-chlorophenyl, 2-chloro-4-fluorophenyl, 2,4,6-trifluorophenyl and 4-bromo-2,5-difluorophenyl.

4. A compound as claimed in claim 3, wherein R is 2,4-dichlorophenyl, 2,4-difluorophenyl or 4-chlorophenyl.

5. A compound as claimed in claim 4, wherein R is 2,4-dichlorophenyl or 2,4-difluorophenyl.

6. A compound as claimed in any one of the preceding claims wherein $R^1$ is —CN, —$CSNH_2$, or —$CONR^2R^3$ wherein either

(a) $R^2$ is H, $CH_3$ or $C_2H_5$, and $R^3$ is H, $C_1$—$C_6$ alkyl, *p*-chlorobenzyl, p-chlorophenethyl, *p*-methylphenethyl, —$CH_2CF_3$, 1-adamantyl, 4-pyridylmethyl, cyclopropyl, carbamoylmethyl, —$CH_2.CH{=}CH_2$, 2-hydroxyethyl, 2-(dimethylamino)ethyl, 2-(methylthio)ethyl, 2-(methylsulphinylethyl), 2-(methylsulphonylethyl), p-chlorophenyl, or 2-(p-chlorophenoxy)ethyl;

(b) $R^2$ and $R^3$ taken together with the nitrogen atom to which they are attached represent a group of the formula:—

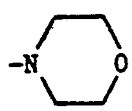 , 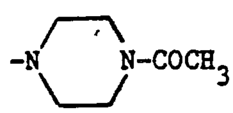 , 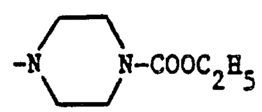 , 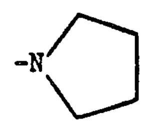 or 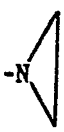 ;

(c) $R^2$ is H and $R^3$ is acetyl, propionyl or p-chlorobenzoyl; or

(d) $R^2$ and $R^3$ are both —$COOCH_3$.

7. A compound as claimed in claim 1 of the formula:—

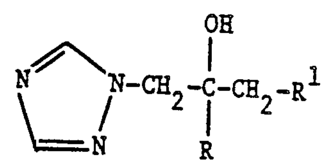

where

R is phenyl optionally substituted by 1 to 3 substituents each independently selected from F, Cl, Br, I, $CF_3$, $C_1$—$C_4$ alkyl and $C_1$—$C_4$ alkoxy, or R is a 5-chloropyrid-2-yl group; and

$R^1$ is —CN or —$CON^2R^3$ where $R^3$ is H, or $C_1$—$C_6$ alkyl, and $R^3$ is H, $C_1$—$C_6$ alkyl, benzyl, —$CH_2CF_3$ or adamantyl, said benzyl group being optionally substituted on its phenyl ring portion by 1 or 2 substituents each independently selected from $C_1$—$C_4$ alkyl, $C_1$—$C_4$ alkoxy, F, Cl, Br, I and $CF_3$, or $R^2$ and $R^3$ taken together with the nitrogen atom to which they are attached represent a group of the formula

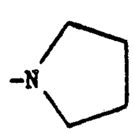 , 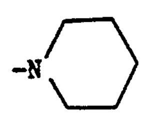 , 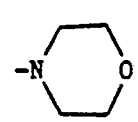 or 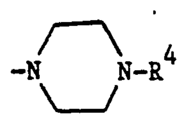

where

$R^4$ is H, $C_1$—$C_4$ alkyl, $C_2$—$C_4$ alkanoyl or ($C_1$—$C_4$ alkoxy)carbonyl;

and their pharmaceutically acceptable salts.

8. A compound as claimed in claim 1 of the formula:—

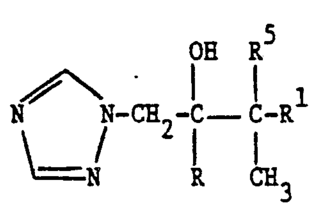

where

R is phenyl optionally substituted by 1 to 3 substituents each independently selected from F, Cl, Br, I, $CF_3$, $C_1$—$C_4$ alkyl and $C_1$—$C_4$ alkoxy, or R is a 5-chloropyrid-2-yl group;

$R^1$ is —CN or —$CONR^2R^3$ where $R^2$ is H or $C_1$—$C_6$ alkyl, and $R^3$ is H, $C_1$—$C_6$ alkyl, benzyl, —$CH_2CF_3$ or adamantyl, said benzyl group being optionally substituted on its phenyl ring portion by 1 or 2 substituents each independently selected from $C_1$—$C_4$ alkyl, $C_1$—$C_4$ alkoxy, F, Cl, Br, I and $CF_3$, or $R^2$ and $R^3$ taken together with the nitrogen atom to which they are attached represent a group of the formula

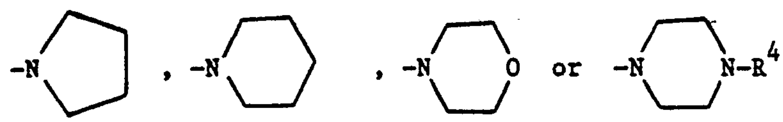

where

$R^4$ is H, $C_1$—$C_4$ alkyl, $C_2$—$C_4$ alkanoyl or ($C_1$—$C_4$ alkoxy)carbonyl; and

$R^5$ is H or $CH_3$;

and their pharmaceutically acceptable salts.

9. A compound as claimed in claim 1 of the formula:—

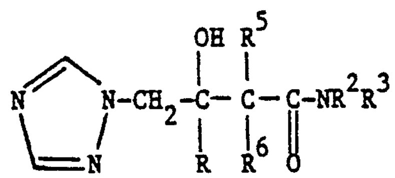

where

R is a phenyl group optionally substituted by 1 to 3 substituents each independently selected from F, Cl, Br, I, $CF_3$, $C_1$—$C_4$ alkyl and $C_1$—$C_4$ alkoxy, or R is a 5-chloropyrid-2-yl group;

$R^5$ and $R^6$ are each independently H or $CH_3$;

and either

(a) $R^2$ is H or $C_1$—$C_4$ alkyl and $R^3$ is $C_2$—$C_4$ alkanoyl or benzoyl, said benzoyl group being optionally substituted by 1 or 2 substituents each independently selected from F, Cl, Br, I, $CF_3$, $C_1$—$C_4$ alkyl and $C_1$—$C_4$ alkoxy; or

(b) $R^2$ and $R^3$ are both ($C_1$—$C_4$ alkoxy)carbonyl; and their pharmaceutically acceptable salts.

10. A compound of the formula (I) as claimed in any one of claims 1 to 8 wherein $R^1$ is —$CONH_2$ or —$CONH(C_1$—$C_4$ alkyl).

11. A compound of the formula (I) as claimed in any one of claims 1 to 6, wherein $R^5$ and $R^6$ are H.

12. A compound of the formula (I) as claimed in claim 1 wherein R is 2,4-dichlorophenyl, $R^5$ and $R^6$ are H, and $R^1$ is —$CONH_2$, —$CONH.CH_3$ or —$CONHC_2H_5$.

13. A pharmaceutical composition comprising a compound of formula (I) as claimed in any one of claims 1 to 12, or a pharmaceutically acceptable salt thereof, together with a pharmaceutically acceptable diluent or carrier.

14. A compound of the formula (I) as defined in any one of claims 1 to 12, or a pharmaceutically acceptable salt thereof, for use as a medicament.

15. The use of a compound of the formula (I) as defined in any one of claims 1 to 12, or a pharmaceutically acceptable salt thereof, for the manufacture of a medicament for use as an anti-fungal agent.

**Claims for the Contracting State: AT**

1. A process for preparing a compound of the formula:—

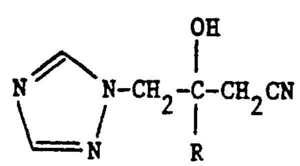

where

R is phenyl optionally substituted by 1 to 3 substituents each independently selected from F, Cl, Br, I, $CF_3$, $C_1$—$C_4$ alkyl and $C_1$—$C_4$ alkoxy, or 5-chloropyrid-2-yl; characterised by reacting a compound of the formula:—

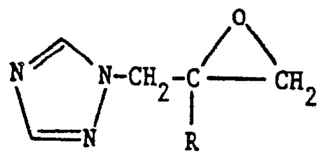 --- (II)

with cyanide ions.

2. A process according to claim 1, characterised in that said cyanide ions are provided by sodium or potassium cyanide.

3. A process for preparing a compound of the formula:—

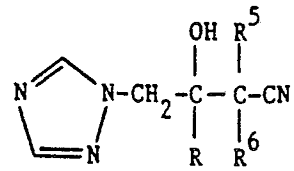

where

R is as defined in claim 1, and $R^5$ and $R^6$ are each H or $CH_3$, characterised by reacting a compound of the formula:—

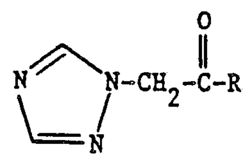 --- (III)

where

R is as defined above, firstly with an anion of the formula:—

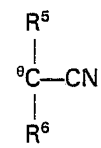

where

$R^5$ and $R^6$ are as defined above, and then with acid.

4. A process according to claim 3, characterised in that the anion is generated by reacting a nitrile of the formula:—

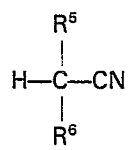

where

$R^5$ and $R^6$ are as defined in claim 3, with n-butyllithium.

5. A process for preparing a compound of the formula:—

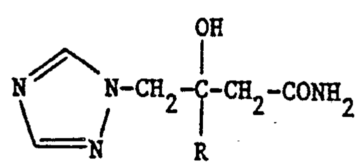

where

R is as defined in claim 1, characterised in that a compound of the formula:—

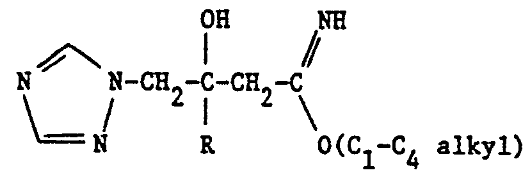 --- (IV)

is reacted with an acid.

6. A process according to claim 5, characterised in that the acid is supplied by using compound (IV) in acid addition salt form, and wherein the reaction is carried by heating the reactants in an organic solvent at up to 180°C.

7. A process for preparing a compound of the formula:—

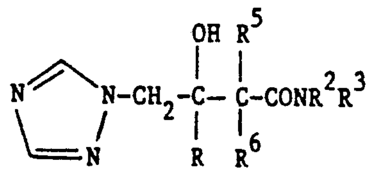

where

R is as defined in claim 1, $R^5$ and $R^6$ are each H or $CH_3$, and either (a) $R^2$ is H or $C_1$—$C_6$ alkyl, and $R^3$ is H, $C_1$—$C_6$ alkyl, benzyl, phenethyl, phenyl, —$CH_2$—$CF_3$, adamantyl, pyridylmethyl, $C_3$—$C_7$ cycloalkyl, carbamoylmethyl, ($C_2$—$C_4$ alkenyl)methyl, 2-hydroxyethyl, 2-(dimethylamino)ethyl, 2-(methylthio)ethyl, 2-(methylsulphinyl)ethyl, 2-(methylsulphonyl)ethyl or 2-phenoxyethyl; said benzyl, phenethyl, phenyl and phenoxy groups being optionally ring-substituted by 1 or 2 substituents each independently selected from $C_1$—$C_4$ alkyl, $C_1$—$C_4$ alkoxy, F, Cl, Br, I and $CF_3$, or (b) $R^2$ and $R^3$ taken together with the nitrogen atom to which they are attached represent a group of the formula:—

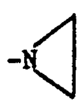 , 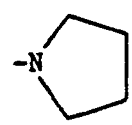 , 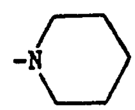 , 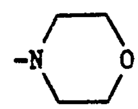 or 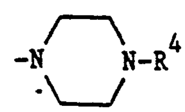

where

$R^4$ is H, $C_1$—$C_4$ alkyl, $C_2$—$C_4$ alkanoyl or ($C_1$—$C_4$alkoxy)carbonyl; characterised by reacting a compound of the formula:—

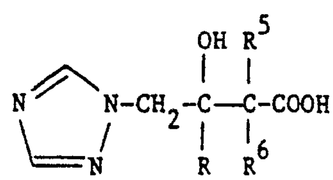 ---(V)

or an acylating derivative thereof, where R, $R^5$ and $R^6$ are as defined above, with a compound of the formula:—

$R^2R^3NH$

where $R^2$ and $R^3$ are as defined above.

8. A process according to claim 7, characterised in that said acylating derivative is an acid chloride or bromide, mixed anhydride, or $C_1$—$C_4$ alkyl, succinimido, phthalimido or benzotriazol-1-yl ester of the acid (V).

9. A process for preparing a compound of the formula:—

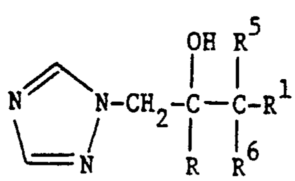

where

$R^1$ is —CONH($C_1$—$C_4$ alkyl) or —CON($C_1$—$C_4$ alkyl)$_2$, and R, $R^5$ and $R^6$ are as defined in claim 3,

characterised by alkylating the corresponding compound in which $R^1$ is $—CONH_2$.

10. A process according to claim 9, characterised in that the alkylation is carried out using the appropriate quantity of a $C_1$—$C_4$ alkyl iodide or bromide in the presence of a base.

11. A process for preparing a compound of the formula:—

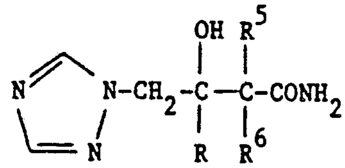

where

R, $R^5$ and $R^6$ are as defined in claim 3, characterised by the controlled hydrolysis of a compound of the formula:—

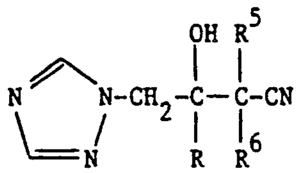

where

R, $R^5$ and $R^6$ are as defined above.

12. A process according to claim 11, characterised in that the hydrolysis is carried out using aqueous sulphuric acid at up to 100°C.

13. A process for preparing a compound of the formula:—

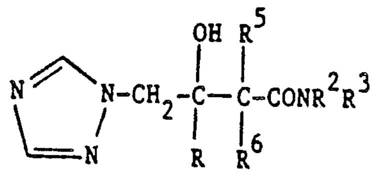

where

R, $R^2$, $R^3$, $R^5$ and $R^6$ are as defined in claim 7, characterised by reacting a compound of the formula:—

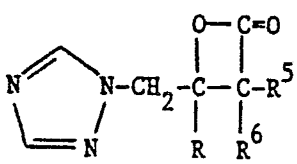

--- (A)

where R, $R^5$ and $R^6$ are as defined above, with an amine of the formula $R^2R^3NH$ where $R^2$ and $R^3$ are as defined above.

14. A process according to claim 13, characterised in that the reaction is carried out in ethanol at about room temperature.

15. A process for preparing a compound of the formula:—

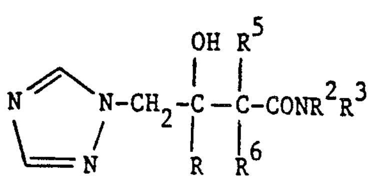

where

R is as defined in claim 1, $R^5$ and $R^6$ are each H or $CH_3$, $R^2$ is H or $C_1$—$C_4$ alkyl, and $R^3$ is $C_2$—$C_4$ alkanoyl or benzoyl, said benzoyl group being optionally substituted by 1 to 2 substituents each independently selected from F, Cl, Br, I, $CF_3$, $C_1$—$C_4$ alkyl and $C_1$—$C_4$ alkoxy, characterised by reacting a compound of the formula:

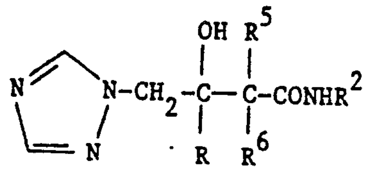 --- (X)

where

R, $R^2$, $R^5$ and $R^6$ are as defined above, with an acid halide or anhydride of the formula:—

$$R^3.X \text{ or } (R^3)_2.O$$

where $R^3$ is as defined above and X is Cl or Br.

16. A process for preparing a compound of the formula:—

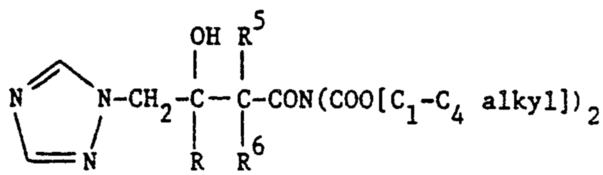

where

R, $R^5$ and $R^6$ are as defined in claim 3, characterised by reacting a compound of the formula:—

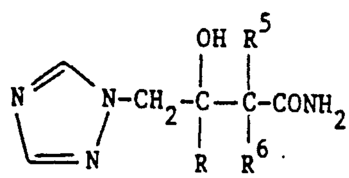 --- (XI)

with at least 2 equivalents of an alkyl haloformate of the formula:—

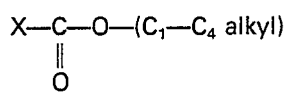

where X is Cl or Br.

17. A process for preparing a compound of the formula:—

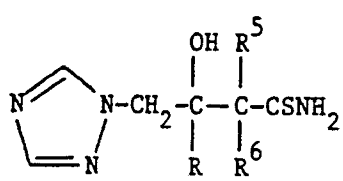

where

R, $R^5$ and $R^6$ are as defined in claim 3, characterised by reacting a compound of the formula:—

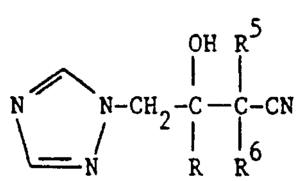

where

R, $R^5$ and $R^6$ are as defined above, with a compound of the formula:—

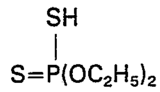

in the presence of water.

18. A process for preparing a compound of the formula:—

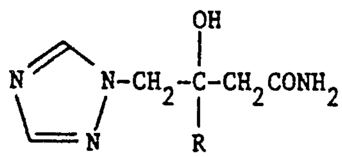

where

R is as defined in claim 1, characterised by reacting a compound of the formula:

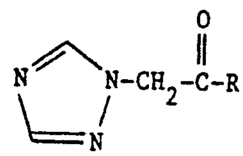

--- (III)

where

R is as defined above, firstly with the product of the reaction of n-butyllithium and bis(trimethylsilyl)-acetamide, and then with an acid.

19. A process for preparing a compound of the formula:—

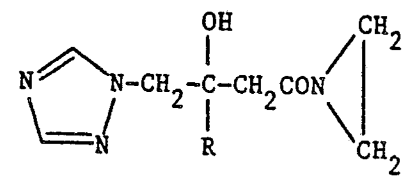

where

R is as defined in claim 1, characterised by reacting a compound of the formula:—

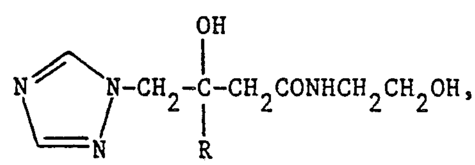

where

R is as defined above, with triphenylphosphine and diethyl azodicarboxylate.

20. A process according to any one of the preceding claims, characterised in that the product of the reaction is converted into a pharmaceutically acceptable acid addition salt by reaction with a suitable acid.

21. Compounds of the formula:—

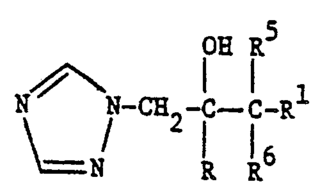

--- (I)

where

R is phenyl optionally substituted by 1 to 3 substituents each independently selected from F, Cl, Br, I, $CF_3$, $C_1$—$C_4$ alkyl and $C_1$—$C_4$ alkoxy, or R is a 5-chloropyrid-2-yl group;

$R^1$ is —CN, —$CSNH_2$, or —$CONR^2R^3$ where either

(a) $R^2$ is H or $C_1$—$C_6$ alkyl, and $R^3$ is H, $C_1$—$C_6$ alkyl, benzyl, phenethyl, phenyl, —$CH_2CF_3$, adamantyl, pyridylmethyl, $C_3$—$C_7$ cycloalkyl, carbamoylmethyl, ($C_2$—$C_4$ alkenyl)methyl, 2-hydroxyethyl, 2-(dimethylamino)ethyl, 2-(methylthio)ethyl, 2-(methylsulphinyl)ethyl, 2-(methylsulphonyl)ethyl or 2-phenoxyethyl;

said benzyl, phenethyl, phenyl and phenoxy group being optionally ring-substituted by 1 or 2 substituents each independently selected from $C_1$—$C_4$ alkyl, $C_1$—$C_4$ alkoxy, F, Cl, Br, I and $CF_3$,

(b) $R^2$ and $R^3$ taken together with the nitrogen atom to which they are attached represent a group of the formula:—

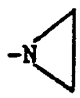 , 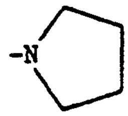 , 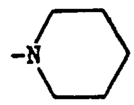 , 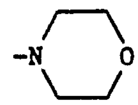 or 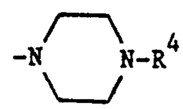

where

$R^4$ is H, $C_1$—$C_4$ alkyl, $C_2$—$C_4$ alkanoyl or ($C_1$—$C_4$ alkoxy)carbonyl;

(c) $R_2$ is H or $C_1$—$C_4$ alkyl and $R^3$ is $C_2$—$C_4$ alkanoyl or benzoyl, said benzoyl group being optionally substituted by 1 or 2 substituents each independently selected from F, Cl, Br, I, $CF_3$, $C_1$—$C_4$ alkyl and $C_1$—$C_4$ alkoxy); or

(d) $R^2$ and $R^3$ are both ($C_1$—$C_4$ alkoxy)carbonyl;

$R^5$ is H or $CH_3$; and

$R^6$ is H or $CH_3$;

and their pharmaceutically acceptable salts.

**Patentansprüche für die Vertragsstaaten: BE CH DE FR GB IT LI LU NL SE**

1. Verbindungen der Formel

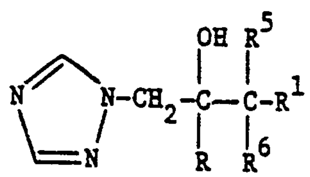 --- (I)

worin

R Phenyl ist, gegebenenfalls substituiert durch 1 bis 3 Substituenten, jeweils unabhängig ausgewählt aus F, Cl, Br, J. $CF_3$, $C_1$—$C_4$-Alkyl und $C_1$—$C_4$-Alkoxy, oder R ist eine 5-Chlorpyrid-2-yl-gruppe; $R^1$ ist —CN, —$CSNH_2$ oder —$CONR^2R^3$, wobei entweder

(a) $R^2$ H oder $C_1$—$C_6$-Alkyl ist und $R^3$ H, $C_1$—$C_6$-Alkyl, Benzyl, Phenethyl, Phenyl, —$CH_2CF_3$, Adamantyl, Pyridylmethyl, $C_3$—$C_7$-Cycloalkyl, Carbamoylmethyl, ($C_2$—$C_4$-Alkenyl)methyl, 2-Hydroxyethyl, 2-(Dimethylamino)ethyl, 2-(Methylthio)ethyl, 2-(Methylsulfinyl)ethyl, 2-(Methylsulfonyl)ethyl oder 2-Phenoxyethyl ist, wobei die Benzyl-, Phenethyl-, Phenyl- und Phenoxygruppe gegebenenfalls durch 1 oder 2-Substituenten, jeweils unabhängig ausgewählt aus $C_1$—$C_4$-Alkyl, $C_1$—$C_4$-Alkoxy, F, Cl, Br, J und $CF_3$, ringsubstituiert ist,

(b) $R^2$ und $R^3$ zusammen mit dem Stickstoffatom, an welches sie gebunden sind, eine Gruppe der Formel

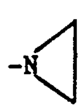 , 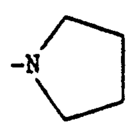 , 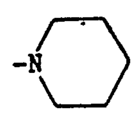 , 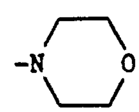 oder 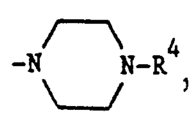 ,

bedeuten, worin $R^4$ H, $C_1$—$C_4$-Alkyl, $C_2$—$C_4$-Alkanoyl oder ($C_1$—$C_4$-Alkoxy)carbonyl ist;

(c) $R^2$ ist H oder $C_1$—$C_4$-Alkyl und $R^3$ ist $C_2$—$C_4$-Alkanoyl oder Benzoyl, wobei die Benzoylgruppe gegebenenfalls durch 1 oder 2 Substituenten, jeweils unabhängig ausgewählt aus F, Cl, Br, J, $CF_3$, $C_1$—$C_4$-Alkyl und $C_1$—$C_4$-Alkoxy, substituiert ist; oder

(d) $R^2$ und $R^3$ sind beide ($C_1$—$C_4$-Alkoxy)carbonyl; $R^5$ ist H oder $CH_3$; und $R^6$ ist H oder $CH_3$; und deren pharmazeutisch annehmbare Salze.

2. Eine Verbindung nach Anspruch 1, worin R Phenyl ist, substituiert durch 1 bis 3 Substituenten, jeweils unabhängig ausgewählt aus F, Cl, Br, J und $CF_3$.

3. Eine Verbindung nach Anspruch 2, worin R 4-Fluorophenyl, 4-Chlorphenyl, 4-Bromphenyl, 4-Jodphenyl, 4-Trifluormethylphenyl, 2-Chlorphenyl, 2,4-Dichlorphenyl, 2,4-Difluorphenyl, 2,5-Difluorphenyl, 2-Fluor-4-chlorphenyl, 2-Chlor-4-fluorphenyl, 2,4,6-Trifluorphenyl und 4-Brom-2,5-difluorphenyl ist.

4. Eine Verbindung nach Anspruch 3, worin R 2,4-Dichlorphenyl, 2,4-Difluorphenyl oder 4-Chlorphenyl ist.

5. Eine Verbindung nach Anspruch 4, worin R 2,4-Dichlorphenyl oder 2,4-Difluorphenyl ist.

6. Eine Verbindung nach irgendeinem der vorhergehenden Ansprüche, worin $R^1$ —CN, —$CSNH_2$ oder —$CONR^2R^3$ ist, worin entweder

(a) $R^2$H, $CH_3$ oder $C_2H_5$ ist und $R^3$ H, $C_1$—$C_6$-Alkyl, p-Chlorbenzyl, p-Chlorphenethyl, p-Methylphenethyl, —$CH_2CF_3$, 1-Adamantyl, 4-Pyridylmethyl, Cyclopropyl, Carbamoylmethyl, —$CH_2.CH{=}CH_2$, 2-Hydroxyethyl, 2-(Dimethylamino)ethyl, 2-(Methylthio)ethyl, 2-(Methylsulfinylethyl), 2-(Methylsulfonylethyl), p-Chlorphenyl oder 2-(p-Chlorphenoxy)ethyl ist;

(b) $R^2$ und $R^3$ zusammen mit dem Stickstoffatom, an welches sie gebunden sind, eine Gruppe der Formel

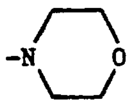 , 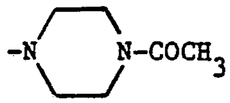 , 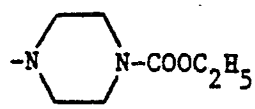 , 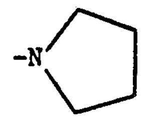 oder 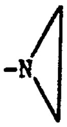

bedeuten;

(c) $R^2$ H ist und $R^3$ Acetyl, Propionyl oder p-Chlorbenzoyl ist; oder

(d) $R^2$ und $R^3$ beide —$COOCH_3$ sind.

7. Eine Verbindung nach Anspruch 1 mit der Formel

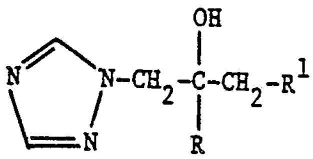

worin

R Phenyl ist, gegebenenfalls substituiert durch 1 bis 3 Substituenten, jeweils unabhängig ausgewählt aus F, Cl, Br, J, $CF_3$, $C_1$—$C_4$-Alkyl und $C_1$—$C_4$-Alkoxy, oder R ist eine 5-Chlorpyrid-2-yl-gruppe; und $R^1$ ist —CN oder —$CONR^2R^3$, worin $R^2$ H oder $C_1$—$C_6$-Alkyl ist und $R^3$ H, $C_1$—$C_6$-Alkyl, Benzyl, —$CH_2CF_3$ oder Adamantyl ist, wobei die Benzylgruppe gegebenenfalls an ihren Phenylringteil durch 1 oder 2 Substituenten, jeweils unabhängig ausgewählt aus $C_1$—$C_4$-Alkyl, $C_1$—$C_4$-Alkoxy, F, Cl, Br, J und $CF_3$, substituiert ist, oder $R^2$ und $R^3$ bedeuten zusammen mit dem Stickstoffatom, an welches sie gebunden sind, eine Gruppe der Formel

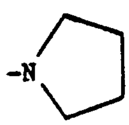 , 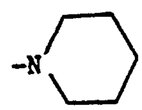 , 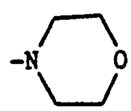 oder 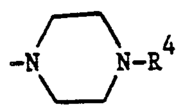

worin

$R^4$ H, $C_1$—$C_4$-Alkyl, $C_2$—$C_4$-Alkanoyl oder ($C_1$—$C_4$-Alkoxy)carbonyl ist; und deren pharmazeutisch annehmbare Salze.

8. Eine Verbindung nach Anspruch 1 mit der Formel

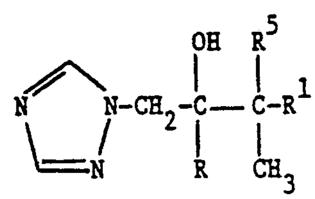

worin

R Phenyl ist, gegebenenfalls substituiert durch 1 bis 3 Substituenten, jeweils unabhängig ausgewählt aus F, Cl, Br, J, $CF_3$, $C_1$—$C_4$-Alkyl und $C_1$—$C_4$-Alkoxy, oder R ist eine 5-Chlorpyrid-2-yl-gruppe; $R^1$ —CN oder —$CONR^2R^3$ ist, worin $R^2$ H oder $C_1$—$C_6$-Alkyl ist und $R^3$ H, $C_1$—$C_6$-Alkyl, Benzyl, —$CH_2CF_3$ oder Adamantyl ist, wobei die Benzylgruppe gegebenenfalls an ihrem Phenylringteil durch 1 oder 2 Substituenten, jeweils unabhängig ausgewählt aus $C_1$—$C_4$-Alkyl, $C_1$—$C_4$-Alkoxy, F, Cl, Br, J und $CF_3$, substituiert ist, oder $R^2$ und $R^3$ bedeuten zusammen mit dem Stickstoffatom, an welches sie gebunden sind, eine Gruppe der Formel

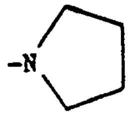 , 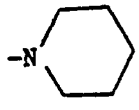 , 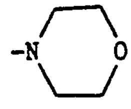 oder 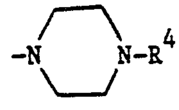

worin

$R^4$ H, $C_1$—$C_4$-Alkyl, $C_2$—$C_4$-Alkanoyl oder ($C_1$—$C_4$-Alkoxy)carbonyl ist; und $R^5$ H oder $CH_3$ ist; und deren pharmazeutisch annehmbare Salze.

9. Eine Verbindung nach Anspruch 1 mit der Formel

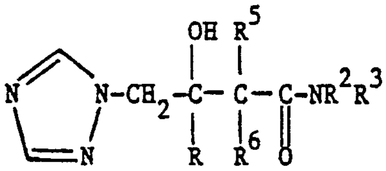

worin

R eine Phenylgruppe ist, gegebenenfalls substiuiert durch 1 bis 3 Substituenten, jeweils unabhängig ausgewählt aus F, Cl, Br, J, $CF_3$, $C_1$—$C_4$-Alkyl und $C_1$—$C_4$-Alkoxy, oder R ist eine 5-Chlorpyrid-2-yl-gruppe;

$R^5$ und $R^6$ sind jeweils unabhängig H oder $CH_3$; und entweder

(a) $R^2$ ist H oder $C_1$—$C_4$-Alkyl und $R^3$ ist $C_2$—$C_4$-Alkanoyl oder Benzoyl, wobei die Benzoylgruppe gegebenenfalls durch 1 oder 2 Substituenten, jeweils unabhängig ausgewählt aus F, Cl, Br, J, $CF_3$, $C_1$—$C_4$-Alkyl und $C_1$—$C_4$-Alkoxy, substituiert ist; oder

(b) $R^2$ und $R^3$ sind beide ($C_1$—$C_4$-Alkoxy)carbonyl; und deren pharmazeutisch annehmbare Salze.

10. Eine Verbindung der Formel (I) nach irgendeinem der Ansprüche 1 bis 8, worin $R^1$ —$CONH_2$ oder —CONH($C_1$—$C_4$-Alkyl) ist.

11. Eine Verbindung der Formel (I) nach irgendeinem der Ansprüche 1 bis 6, worin $R^5$ und $R^6$ H sind.

12. Eine Verbindung der Formel (I) nach Anspruch 1, worin R 2,4-Dichlorphenyl ist, $R^5$ und $R^6$ H sind, und $R^1$ —$CONH.CH_3$ oder —$CONHC_2H_5$ ist.

13. Pharmazeutisch Zusammensetzung, umfassend eine Verbindung der Formel (I) nach irgendeinem der Ansprüche 1 bis 12 oder ein pharmazeutisch annehmbares Salz derselben zusammen mit einem pharmazeutisch annehmbaren Verdünnungsmittel oder Träger.

14. Eine Verbindung der Formel (I) gemäß Definition in irgendeinem der Ansprüche 1 bis 12 oder ein pharmazeutisch annehmbares Salz derselben zur Verwendung als Arzneimittel.

15. Die Verwendung einer Verbindung der Formel (I) gemäß Definition in irgendeinem der Ansprüche 1 bis 12 oder eines pharmazeutisch annehmbaren Salzes derselben zur Herstellung eines Arzneimittels für die Verwendung als antifungales Mittel.

**Patentansprüche für den Vertragsstaat: AT**

1. Verfahren zur Herstellung einer Verbindung der Formel:

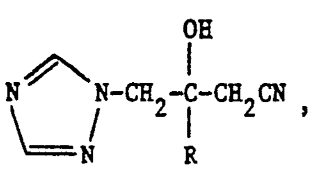

worin

R Phenyl, gegebenenfalls substituiert durch 1 bis 3 Substituenten, jeder unabhängig ausgewählt unter F, Cl, Br, J, $CF_3$, $C_1$—$C_4$-Alkyl und $C_1$—$C_4$-Alkoxy, oder 5-Chlorpyrid-2-yl- ist, dadurch gekennzeichnet, daß eine Verbindung der Formel

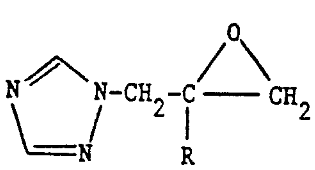

--- (II)

mit Cyanidionen umgesetzt wird.

2. Verfahren nach Anspruch 1, dadurch gekennzeichnet, daß die Cyanidionen durch Natrium- oder Kaliumcyanid geliefert werden.

3. Verfahren zur Herstellung einer Verbindung der Formel

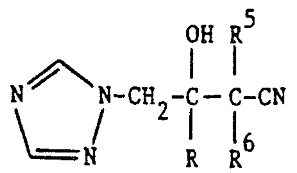

worin

R wie in Anspruch 1 definiert ist und $R^5$ und $R^6$ jeweils H oder $CH_3$ sind, dadurch gekennzeichnet, daß eine Verbindung der Formel

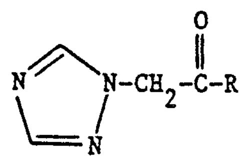 --- (III)

worin R wie oben definiert ist, zuerst mit einem Anion der Formel

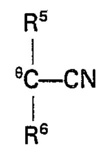

worin

$R^5$ und $R^6$ wie oben definiert sind, und dann mit Säure umgesetzt wird.

4. Verfahren nach Anspruch 3, dadurch gekennzeichnet, daß das Anion durch Umsetzen eines Nitrils der Formel

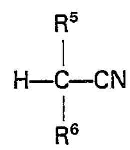

worin

$R^5$ und $R^6$ wie in Anspruch 3 definiert sind, mit n-Butyl-lithium gebildet wird.

5. Verfahren zur Herstellung einer Verbindung der Formel:

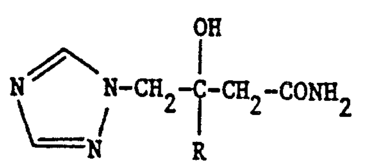

worin

R wie in Anspruch 1 definiert ist, dadurch gekennzeichnet, daß eine Verbindung der Formel

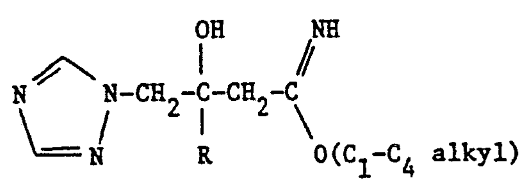 --- (IV)

mit einer Säure umgesetzt wird.

6. Verfahren nach Anspruch 5, dadurch gekennzeichnet, daß die Säure durch Verwendung der Verbindung (IV) in Säureadditionssalzform zugeführt wird, wobei die Umsetzung durch Erwärmen der Reaktionskomponenten in einem organischen Lösungsmittel auf bis zu 180°C erfolgt.

7. Verfahren zur Herstellung einer Verbindung der Formel

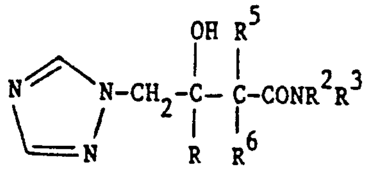

,

worin

R wie in Anspruch 1 definiert ist, $R^5$ und $R^6$ jeweils H oder $CH_3$ sind und entweder

(a) $R^2$ H oder $C_1$—$C_6$-Alkyl und $R^3$ H, $C_1$—$C_6$-Alkyl, Benzyl, Phenethyl, Phenyl, —$CH_2$—$CF_3$, Adamantyl, Pyridylmethyl, $C_3$—$C_7$-Cycloalkyl, Carbamoylmethyl, ($C_2$—$C_4$-Alkenyl)methyl, 2-Hydroxyethyl, 2-(Dimethylamino)ethyl, 2-(Methylthio)ethyl, 2-(Methylsulfinyl)ethyl, 2-(Methylsulfonyl)ethyl oder 2-Phenoxyethyl ist, wobei die Benzyl-, Phenethyl-, Phenyl- und Phenoxygruppen gegebenenfalls substituiert sind durch 1 oder 2 Substituenten, jeweils unabhängig ausgewählt unter $C_1$—$C_4$-Alkyl, $C_1$—$C_4$-Alkoxy, F, Cl, Br, J und $CF_3$, oder

(b) $R^2$ und $R^3$ zusammen mit dem Stickstoffatom, an dem sie hängen, eine Gruppe der Formel bedeuten

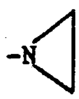

,

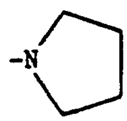

,

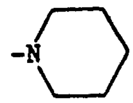

,

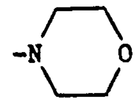

oder

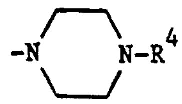

,

worin

$R^4$ H, $C_1$—$C_4$-Alkyl, $C_2$—$C_4$-Alkanoyl oder ($C_1$—$C_4$-Alkoxy)carbonyl ist, dadurch gekennzeichnet, daß eine Verbindung der Formel

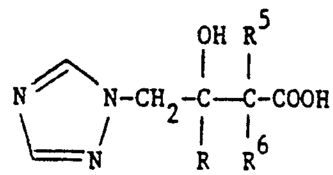

---(V)

oder ein acylierendes Derivat hiervon, worin R, $R^5$ und $R^6$ wie oben definiert sind, mit einer Verbindung der Formel

$$R^2R^3NH,$$

worin

$R^2$ und $R^3$ wie oben definiert sind, umgesetzt wird.

8. Verfahren nach Anspruch 7, dadurch gekennzeichnet, daß das acylierende Derivat ein Säurechlorid oder -bromid, ein gemischtes Anhydrid oder ein $C_1$—$C_4$-Alkyl-, Succinimido-, Phthalimido- oder Benzotriazol-1-ylester der Säure (V) ist.

9. Verfahren zur Herstellung einer Verbindung der Formel

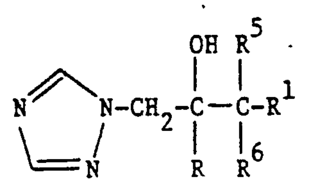

,

worin

$R^1$ —CONH($C_1$—$C_4$-Alkyl) oder —CON($C_1$—$C_4$-Alkyl)$_2$ ist und R, $R^5$ und $R^6$ wie in Anspruch 3 definiert sind, dadurch gekennzeichnet, daß die entsprechende Verbindung, in der $R^1$ —$CONH_2$ ist, alkyliert wird.

10. Verfahren nach Anspruch 9, dadurch gekennzeichnet, daß die Alkylierung unter Verwendung der geeigneten Menge eines $C_1$—$C_4$-Alkyljodids oder -bromids in Gegenwart einer Base erfolgt.

11. Verfahren zur Herstellung einer Verbindung der Formel

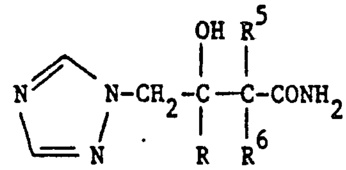

worin
R, $R^5$ und $R^6$ wie in Anspruch 3 definiert sind, dadurch gekennzeichnet, daß eine Verbindung der Formel

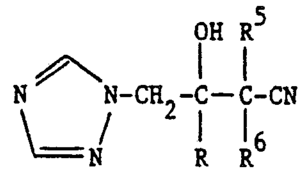

worin
R, $R^5$ und $R^6$ wie oben definiert sind, gesteuert hydrolysiert wird.

12. Verfahren nach Anspruch 11, dadurch gekennzeichnet, daß die Hydrolyse mit wäßriger Schwefelsäure bei bis zu 100°C erfolgt.

13. Verfahren zur Herstellung einer Verbindung der Formel

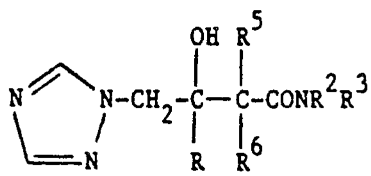

worin R, $R^2$, $R^3$, $R^5$ und $R^6$ wie in Anspruch 7 definiert sind, dadurch gekennzeichnet, daß eine Verbindung der Formel

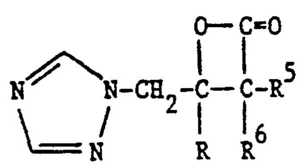

--- (A)

worin R, $R^5$ und $R^6$ wie oben definiert sind, mit einem Amin der Formel $R^2R^3NH$, worin $R^2$ und $R^3$ wie oben definiert sind, umgesetzt wird.

14. Verfahren nach Anspruch 13, dadurch gekennzeichnet, daß die Umsetzung in Ethanol bei etwa Raumtemperatur erfolgt.

15. Verfahren zur Herstellung einer Verbindung der Formel

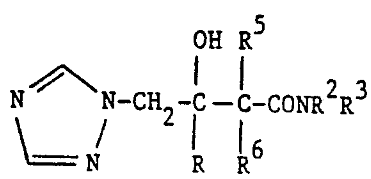

worin
R wie in Anspruch 1 definiert ist, $R^5$ und $R^6$ jeweils H oder $CH_3$ sind, $R^2$ H oder $C_1$—$C_4$-Alkyl ist und $R^3$ $C_2$—$C_4$-Alkanoyl- oder Benzoyl ist, wobei die Benzoylgruppe gegebenenfalls substituiert ist durch 1 oder 2 Substituenten, jeder unabhängig ausgewählt unter F, Cl, Br, J, $CF_3$, $C_1$—$C_4$-Alkyl und $C_1$—$C_4$-Alkoxy, dadurch gekennzeichnet, daß eine Verbindung der Formel

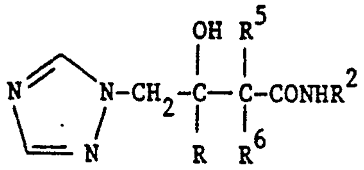

--- (X)

worin
R, $R^2$, $R^5$ und $R^6$ wie oben definiert sind, mit einem Säurehalogenid oder -anhydrid der Formel:

$R^3.X$ oder $(R^3)_2 \cdot O$ ,

worin
$R^3$ wie oben definiert und X Cl oder Br ist, umgesetzt wird.

16. Verfahren zur Herstellung einer Verbindung der Formel

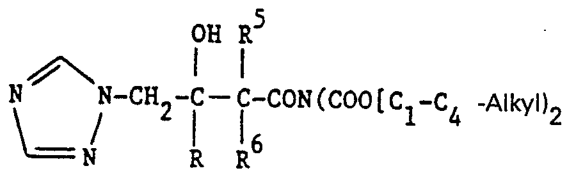

worin
R, $R^5$ und $R^6$ wie in Anspruch 3 definiert sind, dadurch gekennzeichnet, daß eine Verbindung der Formel

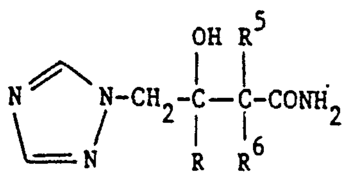

--- (XI)

mit wenigstens 2 Äquivalenten eines Alkylhalogenformiats der Formel

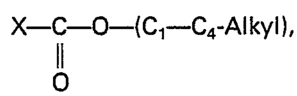

worin
X Cl oder Br ist, umgesetzt wird.

17. Verfahren zur Herstellung einer Verbindung der Formel

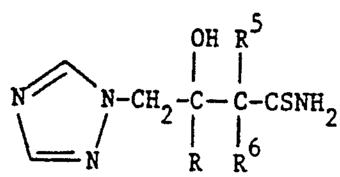

worin
R, $R^5$ und $R^6$ wie in Anspruch 3 definiert sind, dadurch gekennzeichnet, daß eine Verbindung der Formel

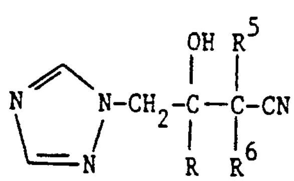

worin
R, $R^5$ und $R^6$ wie oben definiert sind, mit einer Verbindung der Formel

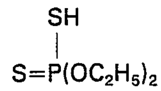

in Gegenwart von Wasser umgesetzt wird.

18. Verfahren zur Herstellung einer Verbindung der Formel:

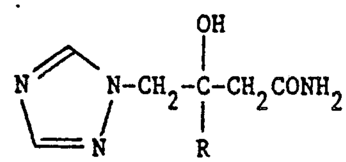

worin

R wie in Anspruch 1 definiert ist, dadurch gekennzeichnet, daß eine Verbindung der Formel

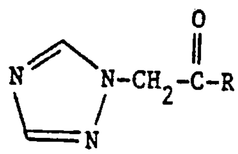

--- (III)

worin

R wie oben definiert ist, zuerst mit dem Produkt der Umsetzung von n-Butyllithium und Bis(trimethylsilyl)acetamid und dann mit einer Säure umgesetzt wird.

19. Verfahren zur Herstellung einer Verbindung der Formel

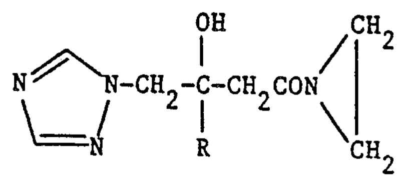

worin

R wie in Anspruch 1 definiert ist, dadurch gekennzeichnet, daß eine Verbindung der Formel

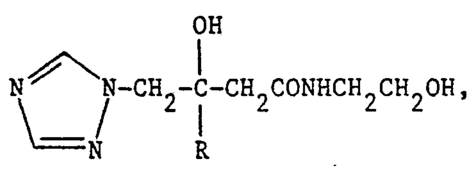

worin

R wie oben definiert ist, mit Triphenylphosphin und Diethylazodicarboxylat umgesetzt wird.

20. Verfahren nach irgendeinem der vorhergehenden Ansprüche, dadurch gekennzeichnet, daß das Produkt der Umsetzung in ein pharmazeutisch annehmbares Säureadditionssalz durch Reaktion mit einer geeigneten Säure umgewandelt wird.

21. Verbindung der Formel

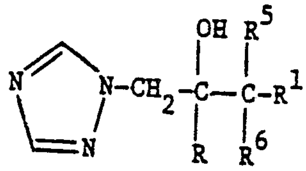

--- (I)

worin

R Phenyl ist, gegebenenfalls substituiert durch 1 bis 3 Substituenten, jeweils unabhängig ausgewählt aus F, Cl, Br, J, $CF_3$, $C_1$—$C_4$-Alkyl und $C_1$—$C_4$-Alkoxy oder R ist eine 5-Chlorpyrid-2-yl-gruppe; $R^1$ ist —CN,

—$CSNH_2$ oder —$CONR^2R^3$, worin entweder

(a) $R^2$ H oder $C_1$—$C_6$-Alkyl ist und $R^3$ H, $C_1$—$C_6$-Alkyl, Benzyl, Phenethyl, Phenyl, —$CH_2CF_3$, Adamantyl, Pyridylmethyl, $C_3$—$C_7$-Cycloalkyl, Carbamoylmethyl, ($C_2$—$C_4$-Alkenyl)methyl, 2-Hydroxyethyl, 2-(Dimethylamino)ethyl, 2-(Methylthio)ethyl, 2-(Methylsulfinyl)ethyl, 2-(Methylsulfonyl)ethyl oder 2-Phenoxyethyl ist, wobei die Benzyl-, Phenethyl-, Phenyl- und Phenoxygruppe gegebenenfalls durch 1 oder 2 Substituenten, jeweils unabhängig ausgewählt aus $C_1$—$C_4$-Alkyl, $C_1$—$C_4$-Alkoxy, F, Cl, Br, J und $CF_3$, ringsubstituiert ist;

(b) $R^2$ und $R^3$ zusammen mit dem Stickstoffatom, an welches sie gebunden sind, eine Gruppe der Formel

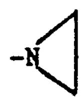 , 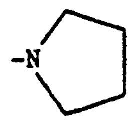 , 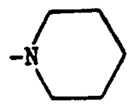 , 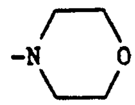 oder 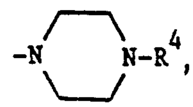 ,

bedeuten, worin

$R^4$ H, $C_1$—$C_4$-Alkyl, $C_2$—$C_4$-Alkanoyl oder ($C_1$—$C_4$-Alkoxy)carbonyl ist;

(c) $R^2$ H oder $C_1$—$C_4$-Alkyl ist und $R^3$ $C_2$—$C_4$-Alkanoyl oder Benzoyl ist, wobei die Benzoylgruppe gegebenenfalls durch 1 oder 2 Substituenten, jeweils unabhängig ausgewählt aus F, Cl, Br, J. $CF_3$, $C_1$—$C_4$-Alkyl und $C_1$—$C_4$-Alkoxy substituiert ist; oder

(d) $R^2$ und $R^3$ beide ($C_1$—$C_4$-Alkoxy)carbonyl sind; $R^5$ H oder $CH_3$ ist; und $R^6$ H oder $CH_3$ ist; und deren pharmazeutisch annehmbare Salze.

**Revendications pour les Etats contractants: BE CH DE FR GB IT LI LU NL SE**

1. Composés de formule:

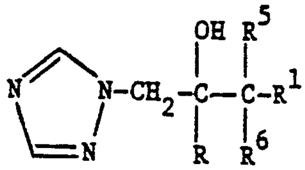 — (I)

dans laquelle R est un groupe phényle facultativement substitué par 1 à 3 substituants choisis, indépendamment, entre F, Cl, Br, I, $CF_3$, un groupe alkyle en $C_1$ à $C_4$ et un groupe alkoxy en $C_1$ à $C_4$, ou bien R est un groupe 5-chloropyrid-2-yle; $R^1$ est un groupe —CN, —$CSNH_2$ ou —$CONR^2R^3$ dans lequel (a) $R^2$ représente H ou un groupe alkyle en $C_1$ à $C_6$ et $R^3$ représente H, un groupe alkyle en $C_1$ à $C_6$, benzyle, phénéthyle, phényle, —$CH_2CF_3$, adamantyle, pyridylméthyle, cycloalkyle en $C_3$ à $C_7$, carbamoylméthyle, (alcényle en $C_2$ à $C_4$)méthyle, 2-hydroxyéthyle, 2-(diméthylamino)éthyle, 2-(méthylthio)éthyle, 2-(méthylsulfinyl)éthyle, 2-(méthylsulfonyl)éthyle ou 2-phénoxyéthyle; lesdits groupes benzyle, phényléthyle, phényle et phénoxy étant facultativement substitués sur le noyau par un ou deux substituants choisis chacun, indépendamment, entre des substituants alkyle en $C_1$ à $C_4$, alkoxy en $C_1$ à $C_4$, F, Cl, Br, I et $CF_3$, (b) $R^2$ et $R^3$ forment, conjointement avec l'atome d'azote auquel ils sont liés, un groupe de formule:

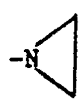 , 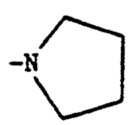 , 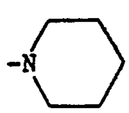 , 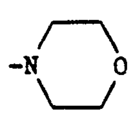 ou 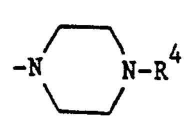

dans laquelle $R^4$ représente H, un reste alkyle en $C_1$ à $C_4$, alcanoyle en $C_2$ à $C_4$ ou (alkoxy en $C_1$ à $C_4$)-carbonyle; (c) $R^2$ représente H ou un groupe alkyle en $C_1$ à $C_4$ et $R^3$ est un groupe alcanoyle en $C_2$ à $C_4$ ou benzoyle, ledit groups benzoyle étant facultativement substitué par 1 ou 2 substituants choisis, indépendamment, entre F, Cl, Br, I, $CF_3$, un substituant alkyle en $C_1$ à $C_4$ et un substituant alkoxy en $C_1$ à $C_4$; ou bien (d) $R^2$ et $R^3$ représentent tous deux un groupe (alkoxy en $C_1$ à $C_4$)-carbonyle; $R^5$ représente H ou $CH_3$; et $R^6$ représente H ou $CH_3$; et leurs sels pharmaceutiquement acceptables.

2. Composé suivant la revendication 1, dans lequel R est un groupe phényle substitué par 1 à 3 substituants choisis chacun, indépendamment, entre des substituants F, Cl, Br, I et $CF_3$.

3. Composé suivant la revendication 2, dans lequel R est un substituant, 4-fluorophényle, 4-chlorophényle, 4-bromophényle, 4-iodophényle, 4-trifluorométhylphényle, 2-chlorophényle, 2,4-dichlorophényle, 2,4-difluorophényle, 2,5-difluorophényle, 2-fluoro-4-chlorophényle, 2-chloro-4-fluorophényle,

2,4,6-trifluorophényle et 4-bromo-2,5-difluorophényle.

4. Composé suivant la revendication 3, dans lequel R est un groupe 2,4-dichlorophényle, 2,4-difluorophényle ou 4-chlorophényle.

5. Composé suivant la revendication 4, dans lequel R est un groupe 2,4-dichlorophényle ou 2,4-difluorophényle.

6. Composé suivant l'une quelconque des revendications précédentes, dans lequel $R^1$ est un groupe —CN, —$CSNH_2$ ou —$CONR^2R^3$ dans lequel

(a) $R^2$ représente H, $CH_3$ ou $C_2H_5$ et $R^3$ représente H, un groupe alkyle en $C_1$ à $C_6$, p-chlorobenzyle, p-chlorophénéthyle, p-méthylphénéthyle, —$CH_2CF_3$, 1-adamantyle, 4-pyridylméthyle, cyclopropyle, carbamoylméthyle, —$CH_2 \cdot CH=CH_2$, 2-hydroxyéthyle, 2-(diméthylamino)éthyle, 2-(méthylthio)éthyle, 2-(méthylsulfinyléthyle), 2-(méthylsulfonyléthyle), p-chlorophényle ou 2-(p-chlorophénoxy)éthyle;

(b) $R^2$ et $R^3$ forment, conjointement avec l'atome d'azote auquel ils sont liés, un groupe de formule:

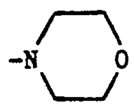 , 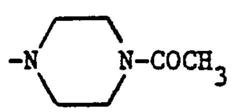 , 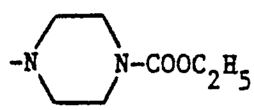 , 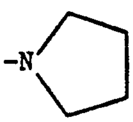 ou 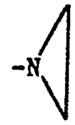 ;

(c) $R^2$ représente H et $R^3$ est un groupe acétyle, propionyle ou p-chlorobenzoyle; ou

(d) $R^2$ et $R^3$ représentent chacun un groupe —$COOCH_3$.

7. Composé suivant la revendication 1, de formule:

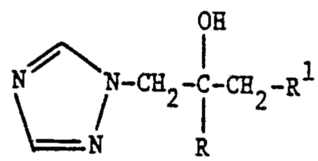

dans laquelle R est un groupe phényle facultativement substitué par 1 à 3 substituants choisis chacun, indépendamment, entre des substituants F, Cl, Br, I, $CF_3$, alkyle en $C_1$ à $C_4$ et alkoxy en $C_1$ à $C_4$, ou bien R est un groupe 5-chloropyrid-2-yle; et $R^1$ est un groupe —CN ou —$CONR^2R^3$ dans lequel $R^2$ représente H ou un groupe alkyle en $C_1$ à $C_6$ et $R^3$ représente H, un groupe alkyle en $C_1$ à $C_6$, benzyle, —$CH_2CF_3$ ou adamantyle, ledit groupe benzyle étant facultativement substitué, sur la portion constituant son noyau phényle, par 1 ou 2 substituants choisis chacun, indépendamment, entre des substituants alkyle en $C_1$ à $C_4$, alkoxy en $C_1$ à $C_4$, F, Cl, Br, I et $CF_3$, ou bien $R^2$ et $R^3$ forment, conjointement avec l'atome d'azote auquel ils sont liés, un groupe de formule:

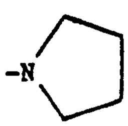 , 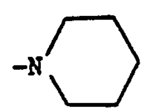 , 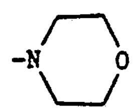 ou 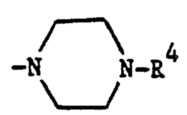

dans laquelle $R^4$ représente H, un groupe alkyle en $C_1$ à $C_4$, alcanoyle en $C_1$ à $C_4$ ou (alkoxy en $C_1$ à $C_4$)-carbonyle; et leurs sels pharmaceutiquement acceptables.

8. Composé suivant la revendication 1, de formule:

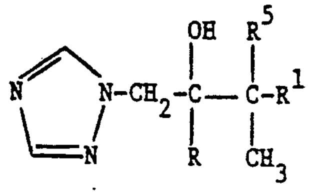

dans laquelle R est un groupe phényle facultativement substitué par 1 á 3 substituants choisis chacun, indépendamment, entre F, Cl, Br, I, un groupe $CF_3$, alkyle en $C_1$ à $C_4$ et alkoxy en $C_1$ à $C_4$, ou bien R est un groupe 5-chloropyrid-2-yle; est un groupe —CN ou —$CONR^2R^3$ dans lequel $R^2$ représente H ou un groupe alkyle en $C_1$ à $C_6$ et $R^3$ représente H, un groupe alkyle en $C_1$ à $C_6$, benzyle, —$CH_2CF_3$ ou adamantyle, ledit groupe benzyle étant facultativement substitué, sur la portion constituant son noyau phényle, par 1 ou 2 substituants choisis chacun, indépendamment, entre un groupe alkyle en $C_1$ à $C_4$, un groupe alkoxy en $C_1$ à $C_4$, F, Cl, Br, I et le groupe $CF_3$, ou bien $R^2$ et $R^3$ forment, conjointement avec l'atome d'azote auquel ils sont liés, un groupe de formule:

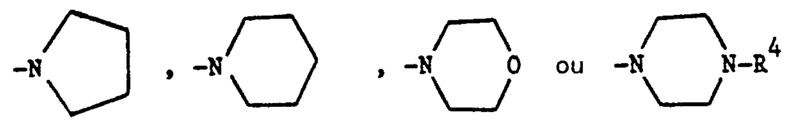

dans laquelle $R^4$ représente H, un groupe alkyle en $C_1$ à $C_4$, un groupe alcanoyle en $C_2$ à $C_4$ ou un groupe (alkoxy en $C_1$ à $C_4$)-carbonyle; et $R^5$ représente H ou un groupe $CH_3$; et leurs sels pharmaceutiquement acceptables.

9. Composé suivant la revendication 1, de formule:

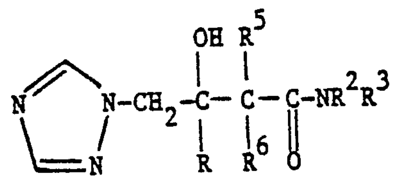

dans laquelle R est un groupe phényle facultativement substitué par 1 à 3 substituants choisis chacun, indépendamment, entre F, Cl, Br, I, un groupe $CF_3$, alkyle en $C_1$ à $C_4$ et alkoxy en $C_1$ à $C_4$, ou bien R est un groupe 5-chloropyrid-2-yle;

$R^5$ et $R^6$ représentent chacun indépendamment H ou un groupe $CH_3$; et

(a) $R^2$ représente H ou un groupe alkyle en $C_1$ à $C_4$ et $R^3$ est un groupe alcanoyle en $C_2$ à $C_4$ ou benzoyle, ledit groupe benzoyle étant facultativement substitué par 1 ou 2 substituants choisis chacun, indépendamment, entre F, Cl, Br, I, $CF_3$, un groupe alkyle en $C_1$ à $C_4$ et un groupe alkoxy en $C_1$ à $C_4$; ou bien

(b) $R^2$ et $R^3$ représentent tous deux un groupe (alkoxy en $C_1$ à $C_4$)-carbonyle; et leurs sels pharmaceutiquement acceptables.

10. Composé de formule (I) suivant l'une quelconque des revendications 1 à 8, dans lequel $R^1$ est un groupe —$CONH_2$ ou —CONH(alkyle en $C_1$ à $C_4$).

11. Composé de formule (I) suivant l'une quelconque des revendications 1 à 6, dans lequel $R^5$ et $R^6$ représentent H.

12. Composé de formule (I) suivant la revendication 1, dans lequel R est un groupe 2,4-dichlorophényle, $R^5$ et $R^6$ représentent H, et $R^1$ est un groupe —$CONH_2$, —$CONH.CH_3$ ou —$CONHC_2H_5$.

13. Composition pharmaceutique comprenant un composé de formule (I) suivant l'une quelconque des revendications 1 à 12, ou un sel pharmaceutiquement acceptable de ce composé, conjointement avec un diluant ou support acceptable du point de vue pharmaceutique.

14. Composé de formule (I) tel que défini dans l'une quelconque des revendications 1 à 12, ou un sel pharmaceutiquement acceptable de ce composé, destiné à être utilisé comme médicament.

15. Utilisation d'un composé de formule (I) tel que défini dans l'une quelconque des revendications 1 à 12 ou d'un sel pharmaceutiquement acceptable de ce composé pour la préparation d'un médicament destiné à être utilisé comme agent anti-fongique.

**Revendications pour l'Etat contractant: AT**

1. Procédé de préparation d'un composé de formule:

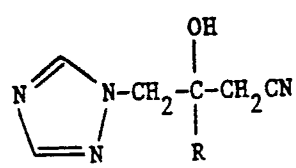

dans laquelle R est un groupe phényle, facultativement substitué par 1 à 3 substituants choisis chacun, indépendamment, entre F, Cl, Br, I, $CF_3$, un groupe alkyle en $C_1$ à $C_4$ et un groupe alkoxy en $C_1$ à $C_4$; ou le groupe 5-chloropyrid-2-yle; caractérisé par la réaction d'un composé de formule:

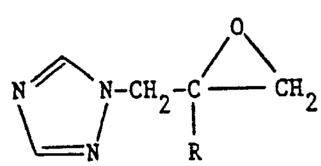

--- (II)

avec des ions cyanure.

2. Procédé suivant la revendication 1, caractérisé en ce que les ions cyanure sont apportés par le cyanure de sodium ou de potassium.

3. Procédé de préparation d'un composé de formule:

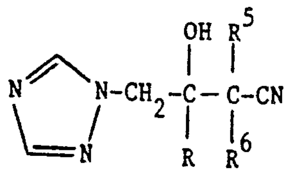

dans laquelle R est tel que défini dans la revendication 1 et $R^5$ et $R^6$ représentent chacun H ou un groupe $CH_3$, caractérisé par la réaction d'un composé de formule:

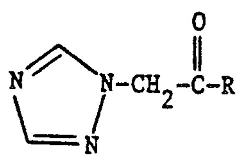

--- (III)

dans laquelle R a la définition donnée ci-dessus, tout d'abord avec un anion de formule:

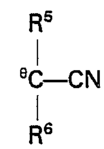

dans laquelle $R^5$ et $R^6$ ont la définition donnée ci-dessus, puis avec un acide.

4. Procédé suivant la revendication 3, caractérisé en ce que l'anion est engendré par réaction d'un nitrile de formule:

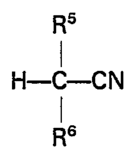

dans laquelle $R^5$ et $R^6$ sont tels que définis dans la revendication 3, avec le n-butyllithium.

5. Procédé de préparation d'un composé de formule:

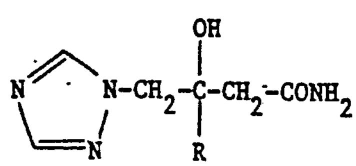

dans laquelle R est tel que défini dans la revendication 1, caractérisé en ce qu'un composé de formule:

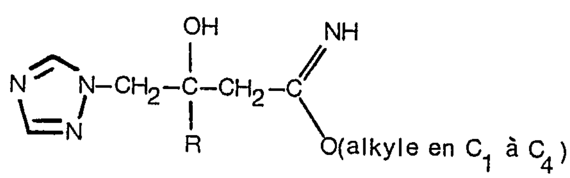

(IV)

est amené à réagir avec un acide.

6. Procédé suivant la revendication 5, caractérisé en ce que l'acide est fourni par l'utilisation du composé (IV) sous la forme d'un sel d'addition d'acide, et la réaction est conduite par chauffage des corps réactionnels dans un solvant organique à une température allant jusqu'à 180°C.

7. Procédé de préparation d'un composé de formule:

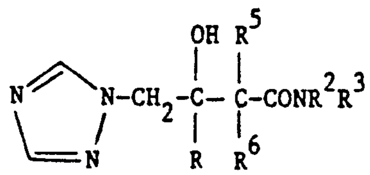

dans laquelle R a la définition donnée dans la revendication 1, $R^5$ et $R^6$ représentent chacun H ou $CH_3$ et (a) $R^2$ représente H ou un groupe alkyle en $C_1$ à $C_6$ et $R^3$ représente H, un groupe alkyle en $C_1$ à $C_6$, benzyle, phénéthyle, phényle, $-CH_2CF_3$, adamantyle, pyridylméthyle, cycloalkyle en $C_3$ à $C_7$, carbamoylméthyle, (alcényle en $C_2$ à $C_4$)-méthyle, 2-hydroxyéthyle, 2-(diméthylamino)éthyle, 2-(méthylthio)-éthyle, 2-(méthylsulfinyl)-éthyle, 2-(méthylsulfonyl)éthyle ou 2-phénoxyéthyle; lesdits groupes benzyle, phényléthyle, phényle et phénoxy étant facultativement substitués sur le noyau par 1 ou 2 substituants sur choisis chacun, indépendamment, entre des substituants alkyle en $C_1$ à $C_4$, alkoxy en $C_1$ à $C_4$, F, Cl, Br, I et $CF_3$; ou bien (b) $R^2$ et $R^3$ forment, conjointement avec l'atome d'azote auquel ils sont liés, un groupe de formule:

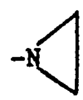 , 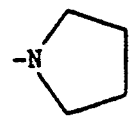 , 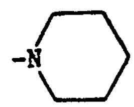 , 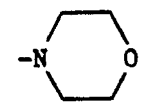 ou 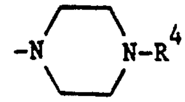

dans laquelle $R^4$ représente H, un groupe alkyle en $C_1$ à $C_4$, alcanoyle en $C_2$ à $C_4$ ou (alkoxy en $C_1$ à $C_4$)-carbonyle; caractérisé par la réaction d'un composé de formule:

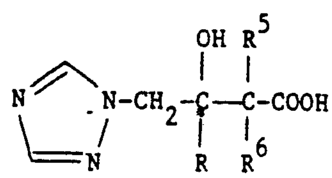 ---(V)

ou d'un dérivé acylant de ce composé où R, $R^5$ et $R^6$ sont tels que définis ci-dessus, avec un composé de formule:

$$R^2R^3NH$$

dans laquelle $R^2$ et $R^3$ sont tels que définis ci-dessus.

8. Procédé suivant la revendication 7, caractérisé en ce que le dérivé acylant est un chlorure ou un bromure d'acide, un anhydride mixte ou un ester d'alkyle en $C_1$ à $C_4$, un succinimido-ester, un phtalimido-ester ou un ester de benzotriazole-1-yle de l'acide (V).

9. Procédé de préparation d'un composé de formule:

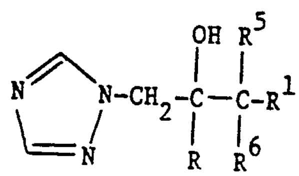

dans laquelle $R^1$ est un groupe $-CONH$(alkyle en $C_1$ à $C_4$) ou $-CON$(alkyle en $C_1$ à $C_4$)$_2$ et R, $R^5$ et $R^6$ sont tels que définis dans la revendication 3, caractérisé par l'alkylation du composé correspondant dans lequel $R^1$ est un groupe $-CONH_2$.

10. Procédé suivant la revendication 9, caractérisé en ce que l'alkylation est effectuée en utilisant la quantité appropriée d'un iodure ou bromure d'alkyle en $C_1$ à $C_4$ en présence d'une base.

11. Procédé de préparation d'un composé de formule:

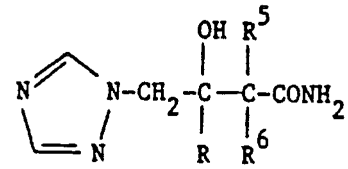

dans laquelle R, $R^5$ et $R^6$ sont tels que définis dans la revendication 3, caractérisé par l'hydrolyse réglé d'un composé de formule

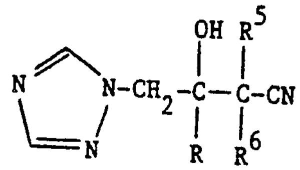

dans laquelle R, $R^5$ et $R^6$ sont tels que définis ci-dessus.

12. Procédé suivant la revendication 11, caractérisé en ce que l'hydrolyse est conduite en utilisant une solution aqueuse d'acide sulfurique à une température allant jusqu'à 100°C.

13. Procédé de préparation d'un composé de formule:

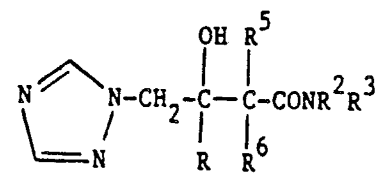

dans laquelle R, $R^2$, $R^3$, $R^5$ et $R^6$ sont tels que définis dans la revendication 7, caractérisé par la réaction d'un composé de formule:

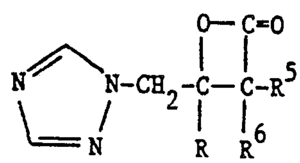 --- (A)

dans laquelle R, $R^5$ et $R^6$ sont tels que définis ci-dessus, avec une amine de formule $R^2R^3NH$ dans laquelle $R^2$ et $R^3$ sont tels que définis ci-dessus.

14. Procédé suivant la revendication 13, caractérisé en ce que la réaction est conduite dans l'éthanol, aux environs de la température ambiante.

15. Procédé de préparation d'un composé de formule:

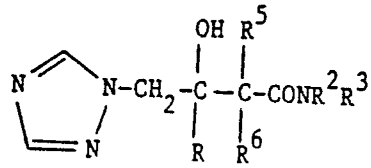

dans laquelle R est tel que défini dans la revendication 1, $R^5$ et $R^6$ représentent chacun H ou $CH_3$, $R^2$ représente H ou un groupe alkyle en $C_1$ à $C_4$ et $R^3$ est un groupe alcanoyle en $C_2$ à $C_4$ ou benzoyle, ledit groupe benzoyle étant facultativement substitué par 1 ou 2 substituants choisis chacun, indépendamment, entre F, Cl, Br, I, le groupe $CF_3$, un groupe alkyle en $C_1$ à $C_4$ et un groupe alkoxy en $C_1$ à $C_4$, caractérisé par la réaction d'un composé de formule:

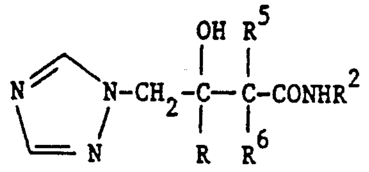 --- (X)

dans laquelle R, $R^2$, $R^5$ et $R^6$ sont tels que définis ci-dessus, avec un halogénure ou anhydride d'acide de formule:

$R^3.X$ ou $(R^3)_2.O$

dans laquelle $R^3$ est tel que défini ci-dessus et X représente Cl ou Br.

16. Procédé de préparation d'un composé de formule:

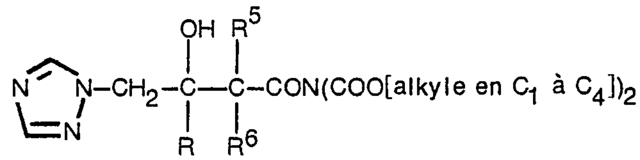

dans laquelle R, $R^5$ et $R^6$ sont tels que définis dans la revendication 3, caractérisé par la réaction d'un composé de formule:

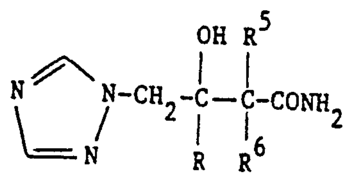 --- (XI)

avec au moins 2 équivalents d'un halogénoformiate d'alkyle de formule:

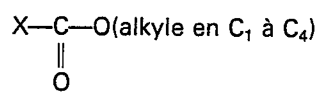

où X représente Cl ou Br.

17. Procédé de préparation d'un composé de formule:

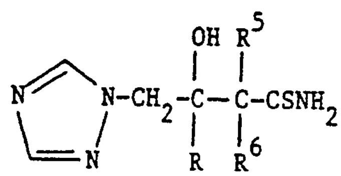

dans laquelle R, $R^5$ et $R^6$ sont tels que définis dans la revendication 3, caractérisé par la réaction d'un composé de formule:

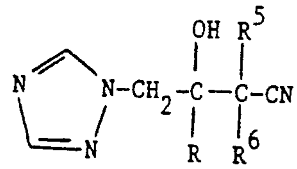

dans laquelle R, $R^5$ et $R^6$ sont tels que définis ci-dessus, avec un composé de formule:

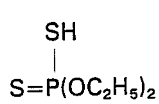

en présence d'eau.

18. Procédé de préparation d'un composé de formule:

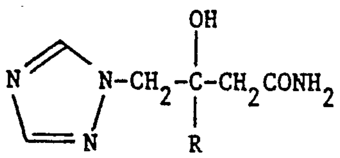

dans laquelle R est tel que défini dans la revendication 1, caractérisé par la réaction d'un composé de formule:

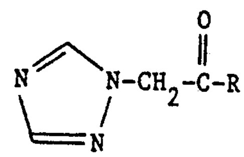
--- (III)

dans laquelle R est tel que défini ci-dessus, tout d'abord avec le produit de réaction du n-butyllithium et du bis(triméthylsilyl)acétamide, puis avec un acide.

19. Procédé de préparation d'un composé de formule:

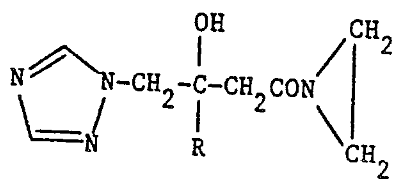

dans laquelle R est tel que défini dans la revendication 1, caractérisé par la réaction d'un composé de formula:

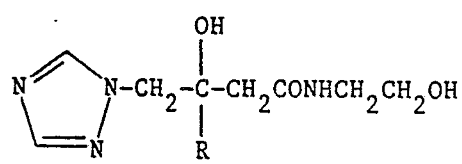

dans laquelle R est tel que défini ci-dessus, avec la triphénylphosphine et l'azodicarboxylate de diéthyle.

20. Procédé suivant l'une quelconque des revendications précédentes, caractérisé en ce que le produit de réaction est converti en un sel d'addition d'acide pharmaceutiquement acceptable par réaction avec un acide convenable.

21. Composés de formule:

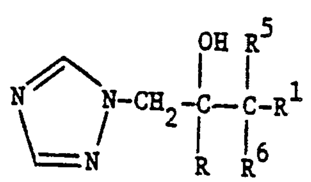
--- (I)

dans laquelle R est un groupe phényle facultativement substitué par 1 à 3 substituants choisis, chacun, indépendamment, entre F, Cl, Br, I, le groupe $CF_3$, un groupe alkyle en $C_1$ à $C_4$ et alkoxy en $C_1$ à $C_4$, ou bien R est un groupe 5-chloropyrid-2-yle; $R^1$ est un groupe —CN, —$CSNH_2$ ou un groupe —$CONR^2R^3$ dans lequel (a) $R^2$ représente H ou un groupe radical alkyle en $C_1$ à $C_6$ et $R^3$ représente H, un groupe radical alkyle en $C_1$ à $C_6$, benzyle, phénéthyle, phényle, —$CH_2CF_3$, adamantyle, pyridylméthyle, cycloalkyle en $C_3$ à $C_7$, carbamoylméthyle, (alcényle en $C_2$ à $C_4$)-méthyle, 2-hydroxyéthyle, 2-(diméthylamino)-éthyle, 2-(méthylthio)-éthyle, 2-(méthylsulfinyl)éthyle, 2-(méthylsulfonyl)-éthyle ou 2-phénoxyéthyle; lesdits groupes benzyle, phényléthyle, phényle et phénoxy étant facultativement substitués sur le noyau par 1 ou 2

substituants choisis chacun, indépendamment, entre des substituants alkyle en $C_1$ à $C_4$, alkoxy en $C_1$ à $C_4$, F, Cl, Br, I et $CF_3$, (b) $R^2$ et $R^3$ forment, conjointement avec l'atome d'azote auquel ils sont liés, un groupe de formule:

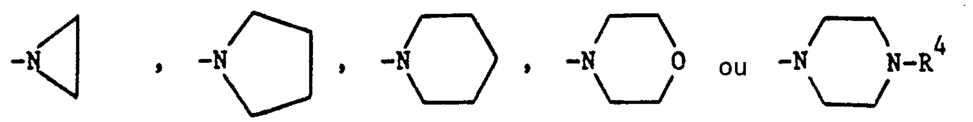

dans laquelle $R^4$ représente H, un groupe alkyle en $C_1$ à $C_4$, alcanoyle en $C_2$ à $C_4$ ou (alkoxy en $C_1$ à $C_4$)-carbonyle; (c) $R^2$ représente H ou un groupe alkyle en $C_1$ à $C_4$ et $R^3$ représente un groupe alcanoyle en $C_2$ à $C_4$ ou benzoyle, ledit groupe benzoyle étant facultativement substitué par 1 ou 2 substituants choisis chacun, indépendamment, entre F, Cl, Br, I, le groupe $CF_3$, un substituant alkyle en $C_1$ à $C_4$ et un group alkoxy en $C_1$ à $C_4$; ou bien (d) $R^2$ et $R^3$ représentent tous deux un groupe (alkoxy en $C_1$ à $C_4$)-carbonyle; $R^5$ représente H ou $CH_3$; et $R^6$ représente H ou $CH_3$; et leurs sels pharmaceutiquement acceptables.